# EUROPEAN PATENT SPECIFICATION

(11) **EP 2 739 296 B1**
(45) Date of publication and mention of the grant of the patent: **18.11.2015**
(21) Application number: 12758634.5
(22) Date of filing: 26.07.2012
(51) Int. Cl.: A61K 36/45, A61P 17/00, A61P 17/18, A61K 8/97, A61Q 5/00, A61Q 19/08, A61Q 19/00, A01H 4/00

(54) **PLANT MATERIAL FROM OXYDENDRUM ARBOREUM FOR COSMETIC USE**
PFLANZLICHES MATERIAL AUS OXYDENDRUM ARBOREUM ZUR KOSMETISCHEN VERWENDUNG
MATIERE VEGETALE EXTRAITE DE L'ARBRE OXYDENDRUM ARBOREUM ET UTILISEE EN COSMETIQUE

(30) Priority: 28.07.2011 FR 1156933
(43) Date of publication of application: 11.06.2014
(73) Proprietor: Sederma, 78610 Le Perray en Yvelines (FR)
(72) Inventor: FOURNIAL, Arnaud, F-75016 Paris (FR); GRIZAUD, Claire-Marie, B-1180 Uccle (BE); MONDON, Philippe, F-92120 Montrouge (FR)
(86) International application number: PCT/IB2012/053821
(87) International publication number: WO 2013/014640

(56) References cited:
- WO-A1-89/04599
- BELKIN M ET AL: "Tumor-damaging capacity of plant materials. II. Plants used as diuretics", JOURNAL OF THE NATIONAL CANCER INSTITUTE 1952, vol. 13, no. 3, 1952, pages 741-744, XP009156901, ISSN: 0027-8874 & BELKIN M ET AL: "TUMOR-DAMAGING CAPACITY OF PLANT MATERIALS I. PLANTS USED AS CATHARTICS", JOURNAL OF THE NATIONAL CANCER INSTITUTE, OXFORD UNIVERSITY PRESS, GB, vol. 13, 7 April 1952 (1952-04-07), pages 139-155, XP001180783, ISSN: 0027-8874
- DATABASE WPI Week 200911 Thomson Scientific, London, GB; AN 2009-B39292 XP002670568, & CN 101 306 099 A (YE M) 19 November 2008 (2008-11-19)
- CAFFERTY STEVE ET AL: "Typification of Linnaean plant names in Ericaceae.", TAXON, vol. 51, no. 4, November 2002 (2002-11), pages 751-753, XP009156899, ISSN: 0040-0262
- CORNELIA SCHÜRCH ET AL: "Potential of plant cells in culture for cosmetic application", PHYTOCHEMISTRY REVIEWS, vol. 7, no. 3, 1 December 2007 (2007-12-01), pages 599-605, XP055043814, ISSN: 1568-7767, DOI: 10.1007/s11101-007-9082-0

## Description

### TECHNICAL FIELD

The invention relates to a new material of plant origin, a topical composition comprising same, and a topical use for improving the appearance and general condition of the skin, mucous membranes and appendages of said material and of said composition.

The present invention concerns more generally the cosmetics, pharmaceutical and cosmeceutical industries, that produce and/or use products for preventing and/or treating disorders and pathologies of skin, scalp, mucous membranes and appendages (such as body hair, eyelashes, eyebrows, nails or hair) of mammals, animals or humans.

These industries are in increased demand for new products, particularly in demand for new active ingredients that are derived from plants because they allow to combine efficiency, limitation of the risk of irritation and allergies, decrease of side effects, biodegradability, with opportunities of labeling / certifications and suitability with a sustainable development and/or fair trade approach.

The present invention aims to meet this demand.

### SUMMARY OF THE INVENTION

"Material of plant origin" means according to the invention any material from plant that may be in any form including, but not limited to, the whole plant, a dried plant, a ground plant, a chopped plant, one or several parts thereof, including, but not limited to, seeds, needles, leaves, roots, bark, cones, stems, rhizomes, callus cells, protoplasts, organs and organ systems, and meristems, or components and/or constituents found in, or isolated from, the material of plant origin, and/or portions of the plant, or extracts derived either directly or synthetically from the plant, or any combinations thereof. It is to be understood that "material of plant origin" also includes an ingredient, component, constituent pure or semi-pure, or an extract or derived solid or liquid, from the material of plant origin.

The material of plant origin covers, according to the invention, in particular a plant extract obtained by the usual techniques, by solvent extraction of the whole plant or specific parts of the plant, by any technique as steeping, simple decoction, lixiviation, extraction under reflux, supercritical fluid extraction, extraction by means of ultrasound or microwaves, or using countercurrent technology, this list being not restrictive. Extraction solvents can be chosen among water, propylene glycol, butylene glycol, glycerin, PEG-6 Caprylic/capric glycerides, polyethylene glycol, methylic and/or ethylic diglycol ethers, cyclic polyols, ethoxylated or propoxylated diglycols, alcohols (methanol, ethanol, propanol, butanol), or any mixture of those solvents.

The material of plant origin according to the invention is obtained by *in vitro* plant culture, more particularly by plant cell culture or by tissue culture from cellular or tissular lines from different plant organs.

The obtaining of materials of plant origin by *in vitro* culture presents numerous advantages over the agro-industrial path (plant culture in open fields and subsequent extraction in factories). The obtained materials are free of toxic substances (herbicides, pesticides, fertilizers, heavy metals and other contaminants, such as those that may come from plant parasite). Moreover, the strict control of *in vitro* culture conditions reduces the risk of spontaneous variation of the strain profile and ensures a reproducible secondary metabolites profile that correspond to the molecules of interest sought, contrary to the culture in an open field where there is the problem of variability due to weather and geography. Furthermore, this technology overcomes obstacles such as the natural biological cycle of the plant and the seasonality of production of the secondary metabolites, thus allowing a better safety and supply rapidity. In addition, the environmental impact is minimal avoiding consumption of arable lands and soils pollution. Furthermore, biodiversity is preserved since it takes only one plant or even a seed to initiate an *in vitro* culture. Finally, this technology offers the ability to direct cellular metabolism toward the production of molecules of interest (elicitation of the cultures) and to achieve some controlled and relatively rapid protocols in order to maximize yields.

The expression "improving the appearance and general condition of the skin, mucous membranes, and appendages", means according to the invention acting both at preventive and at curative levels on disorders and pathologies, signs of a degradation, including fighting against aging, and/or against the imperfections of the skin, mucous membranes or skin appendages.

"Fighting against aging" means preventing and/or limiting the appearance of signs of skin aging such as fine lines, wrinkles and dry skin, loss of tone, elasticity, etc.. It means, when the skin is already showing signs of skin aging, correcting and/or reducing these signs of aging. Aging also affects the mucous membranes and skin appendages such as hair, body hair (including eyebrows and eyelashes) and nails, and the signs of aging can then be very various but no less annoying.

"Fighting against the imperfections of the skin" means preventing and/or limiting the appearance of skin imperfections such as discontinuities in texture or tone, feelings deterioration of skin, mucous membranes or skin appendages such as tightness, dryness, itching. Also it means, when the skin has already imperfections, treating and/or reducing these imperfections.

By "disorders and pathologies", signs of degradation of the appearance and general condition of the skin, mucous membranes or skin appendages, it is understood according to the invention:
- Disorders associated with an increase of MMPs, a decrease in collagen, an increase of glycation, an increase in free radicals, of inflammation, a degradation of extracellular matrix, including:
   o the appearance and development of textural discontinuities such as fine lines and wrinkles, including the number and depth of forehead wrinkles, wrinkles of crow's feet, fine lines and wrinkles inter eyebrows, wrinkles below of the eye and at the corner of the mouth, the folds of the neck;
   o the sagging of cutaneous and subcutaneous tissue, the sagging of the skin of the neck, a withered and flaccid skin, also on the forearms, and on thighs;
   ∘ the loss of firmness, tone and skin elasticity, the ptosis, the atony of skin texture, and skin anisotropy;
   ∘ the variation in the thickness of the epidermis and/or of the dermis, the skin atrophy;
   ∘ the loss of watertight integrity, the loss of skin resistance to deformation;
   ∘ the appearance of bags;
   ∘ the dry and rough appearance of the skin, the roughness, the loss of skin smoothness, the change in skin texture;
   ∘ the dry skin associated in particular with a reduction in sebaceous and/or an increase in insensible water loss of the skin, xerosis;
   ∘ the keratoses, abnormal differentiation, hyperkeratinisation, elastosis;
- Disorders associated with an increase in pigmentation and inflammation, including:
   ∘ the change of the complexion of the skin, the reduction of clarity and brightness of the complexion, the dullness, the loss of uniformity of skin tone, the pigmentation changes (hypo and hyper pigmentation), the appearance of colored or dark spots, of local redness;
   ∘ the pigment spots, hyperkeratosis spots, sunspots visible under UV, lentigines, age spots;
   ∘ an increase in vascular signs (appearance of telangiectasias, angiomas);
- Disorders associated with an increase in free radicals and inflammation, including:
   ∘ the disruption of the microcirculation of the skin and of underlying tissues close to the skin;
   ∘ the appearance of irritated areas;
- Disorders associated with a combination of these factors, including:
   ∘ increased desquamation;
   ∘ the change in pore size, enlarged pores;
   ∘ the change of pH;
   ∘ the change in color, texture and the hair loss, a dull hair, altered hair;
   ∘ an unbalanced scalp;
   ∘ brittle nails;
   ∘ symptoms due to menopause;
   ∘ and other histological changes in the stratum corneum, the dermis and epidermis.
The present invention provides a material of plant origin wherein it is obtained from the genus *Oxydendrum* and in particular of the species *Oxydendrum arboreum* (or *Oxydendron arboreum* in French).

The *Oxydendrum arboreum* is a small slow-growing tree, of dark green foliage, yellow and red in autumn. White flowers in long panicles stand at the tips of branches. It is found in the mountainous forests of the eastern United States.

The tests detailed below show that a material of plant origin obtained from *Oxydendrum arboreum* has an anti-radical and anti-oxidant activity, an anti-inflammatory and anti-glycation activity, effects on extracellular matrix (in particular anti-MMP1, anti-MMP2, anti-MMP9, and increased synthesis of collagen I), and effects on pigmentation (in particular anti-melanogenesis) that make the material be an active ingredient component useful for improving the general state of skin, mucous membranes or appendages, and for fighting against aging or imperfections of the skin, mucous membranes or skin appendages. The material can act preventively or curatively.

Skin, mucous membranes, and appendages age chronologically, hormonally, actinically (photodamage) as well as because of environmental stress. It is to be understood that chronological aging represents the structural, functional, and metabolic changes of the skin that are equivalent to the degenerative changes and due to age in other organs of the body, while the photo-aging is a separate process that largely involves damages to collagen and elastin fibers of the skin and is due to environmental exposure, such as sunlight. During the aging process, the complexion of the skin, that is to say the color and appearance of the skin, is slowly deteriorating. The solar elastosis, an imperfect synthesis of collagens, a disturbance of skin pigmentation or a disturbance of blood supply ... with age, skin loses its brightness, complexion becomes dull, redness and spots appear.

Skin aging is associated with an increase of the number and of the depth of wrinkles, skin folds that are the consequences of the degradation of macromolecules of the dermis such as collagen and elastin. Some changes observed during chronological aging are similar to those shown in the photo-induced aging: overproduction of free radicals, resulting in an increased activity of certain enzymes involved in the degradation of the dermal extracellular matrix, the matrix metalloproteinases (MMPs); reduced presence of dermal macromolecules such as collagens and their degradation.

Each body cell has its own silent network of defense against the disease, the environmental damages and the aging. Part of this network is fighting destructive fragments called free radicals. Just as a car consumes fuel and generates pollution, it is the same for the body cells that produce energy from food and oxygen but leave behind pollutants: free radicals.

Another theory on chronological aging says that aging results from the accumulation of cellular damages caused by excess of free radicals generated during oxidative metabolism. Cellular damages associated with these free radicals are materialized-by many DNA mutations, proteins oxidation and oxidation of lipid membranes, resulting in an alteration of transmembrane signaling. The whole contributs to the overproduction and over-activation of matrix metalloproteinases. The photo-induced aging includes all the phenomena of cellular senescence and of degradation of the dermis proteins. But they are exacerbated in the limbs in contact with the environment and especially under the action of ultraviolets.

Premature aging of the skin also results in an increased redness. Originally circulatory, this phenomenon manifests itself by redness on the cheekbones, nose ... (dilated blood vessels). Before final installation of rosacea, erythrose is an early warning which is manifested by diffuse redness on the surface of skin, but without permanent dilation of micro vessels (telangiectasia). Over the years erythrose settles in rosacea with irreversible redness often localized on cheeks, cheekbones, chin and the presence of telangiectasia purplish red. UV exposure is an aggravating cause for erythrose and adds to the relaxation of capillary walls associated with age: rosacea settles over the long term. With skin photo-aging, blood vessels undergo pronounced changes: induction of erythema, skin inflammation, vascular leakage, edema.... The photo-induced disruption at the micro-capillary (vascular) level is essentially linked to an inflammatory process.

The material of plant origin obtained from *Oxydendrum arboreum* is in particular an extract obtained by *in vitro* plant culture using a technology and a method for obtaining that are presented in more detail thereafter; said material being a cell extract. The invention relates more particularly to an extract obtained by *in vitro* culture of undifferentiated plant cells and particularly from the aerial parts, *i.e.* leaves and stems of *Oxydendrum arboretum.*

The material of the present invention can be used as active ingredient.

An active ingredient is a composition according to the invention containing the material of plant origin of the invention solubilized in a matrix forming a physiologically acceptable medium. This active ingredient is intended for cosmetic, pharmaceutical or cosmeceutical industries, including the cosmetic industry for the preparation of cosmetics, creams, gels, etc.

The ingredient can be called "topical active ingredient" in that its destination is a topical application. "Topical application" means an application that is intended to act where it is applied: skin, mucous membranes, skin appendages. A topical application can particularly be cosmetic, pharmaceutical or cosmeceutical.

In particular the material of the present invention can be used as a cosmetic active ingredient for improving the appearance and general condition of the skin, of mucous membrane, and of skin appendages, and in particular for fighting against aging and/or imperfections of the skin, of the mucous membranes, or of appendages.

The present invention also provides a topical composition characterized in that it contains the material of the invention. The material of the invention can be used pure or diluted in a physiologically acceptable medium forming an excipient or matrix.

The present invention also relates to a topical composition, including cosmetic, pharmaceutical or cosmeceutical, characterized in that it contains, in a physiologically acceptable medium, as an active ingredient, an effective amount of a material of plant origin of a plant of the genus *Oxydendrum*, and more particularly of the species *Oxydendrum arboreum* (also called *Oxydendron arboreum* in French). According to other advantageous features of the present invention, the composition of the invention can contain one or more additional active ingredient, in order to preferably provide an end product with a wider range of properties. Additional active ingredients can be for example selected from whitening agents, anti-redness, UV sunscreens, moisturizing, humectant, exfoliating, anti-aging, anti-wrinkles and anti-fine lines, thinning, volumizing, improving the elastic properties, anti-acne, anti-inflammatory, anti-oxidant, anti-radical, anti-glycant, propigmenting or depigmenting, depilatories, anti-growth or promoting hair growth, peptides, vitamins agents etc. These active ingredients can be obtained from plant materials such as plant extracts or products of plant culture or fermentation.

More specifically, in a topical composition, the extract of plant origin according to the invention can be combined with at least one of the compounds selected from the compounds of the B3 vitamin compounds such as niacinamide and tocopherol, retinoid compounds such as retinol, hexamidine, α-lipoic acid, resveratrol or DHEA, peptides, including N-acetyl-Tyr-Arg-O-hexadecyl, Pal-VGVAPG (SEQ ID NO: 1), Pal-KTTKS (SEQ ID NO: 2), Pal-GHK, Pal-KMO2K and Pal-GQPR (SEQ ID NO: 3), which are conventional active ingredients used in topical cosmetic or dermo-pharmaceutical compositions.

The present invention will be better understood in light of the following description.

### DETAILED DESCRIPTION

### Composition preparation

The expression "physiologically acceptable medium" means according to the present invention, without limitation, an aqueous or hydro-alcoholic solution, a water-in-oil emulsion, an oil-in-water emulsion, a micro-emulsion, an aqueous gel, an anhydrous gel, a serum, a dispersion of vesicles or a powder.

"Physiologically acceptable" means that the compositions are suitable for topical or transdermal use, in contact with mucous membranes, appendages (nails, hair, body hair), scalp and skin of mammals, particularly human, without risk of toxicity, incompatibility, instability, allergic response, and others. The "physiologically acceptable medium" forms what is commonly called the excipient of the composition.

The effective amount of the material of plant origin of the present invention, that is to say its dosage, depends on the destination of the topical composition, cosmetic, pharmaceutical or cosmeceutical.

The cosmetic, pharmaceutical or cosmeceutical effective quantity according to the invention, to administer to treat a disorder or a pathology, and its dosage, depends on various factors such as the age, the state of the patient, the severity of the disorder or condition and the mode of administration. An effective amount means a not toxic amount enough to achieve the desired effect.

In a cosmetic composition for implementing the present invention, the material obtained from *Oxydendrum arboretum*, to be present in an effective amount, is generally present in an amount ranging from 0.00001% to 90%, from 0.0001% to 25%, and from 0.001% and 10% of the total weight of the composition, depending on the destination of the composition and the desired effect more or less pronounced.

All percentages and ratios used herein are by weight of the total composition and all measurements are made at 25°C unless it is otherwise specified.

The choice of excipient in the composition is made according to the constraints of the material of *Oxydendrum arboreum* (stability, solubility, etc.), and if necessary according to the dosage form intended further for the composition.

The material of the invention may be incorporated into the composition by means of an aqueous solution, or be dissolved by the usual physiologically acceptable solubilizers, for example and without limiting to this list: ethanol, propanol, isopropanol, propylene glycol, glycerin, butylene glycol, or polyethylene glycol or any combination thereof. It may also be interesting to solubilize the extract with emulsifiers. A powder medium can also be used.

The compositions for the present invention are generally prepared by conventional methods well known to one skilled in the art for making topical and oral compositions and injection compositions. Such methods may involve a mixture of ingredients in one or more steps to obtain a uniform state, with or without heating, cooling, etc.

The different galenic forms that may contain the material of the invention can be all forms i.e. creams, lotions, milks or creams ointments, gels, emulsions, dispersions, solutions, suspensions, cleansers, foundations, anhydrous preparations (sticks in particular lip balm, body and bath oils), shower and bath gels, shampoo and hair care lotions, milks or creams for skin care or hair, cleansing lotions or milks, sunscreen lotions, milks or creams, artificial tanning lotions, milks or creams, pre shave, shaving or aftershave creams, foams, gels or lotions, makeup, lipstick, mascara or nail polish, skin essences, serums, adhesive or absorbent materials, transdermal patches, or emollient powders, lotions, milks or creams, sprays, body and bath oils, foundation basis, ointment, emulsion, colloid, compact suspension or solid, pencil, sprayable formulation, brushable, blush, red, eyeliner, lipliner, lip gloss, face or body powder, styling gels or mousses, nail conditioning, lip balms, skin conditioners, moisturizers, lacquers, soaps, exfoliants, astringents, depilatories agents, permanent waving solutions, antidandruff formulations, antiperspirant or antiperspirant compositions, including sticks, "roll-on" deodorants, air fresheners, sprays for the nose and etc. These compositions may also be in the form of lipsticks intended either to color the lips or to prevent them from chapping, or makeup for eyes, eyes-shadows and foundations for the face. The compositions for the invention can include cosmetics, personal care products and pharmaceutical preparations. A composition in the form of foam or in the form of aerosol compositions also comprising a pressurized propellant can be considered.

The compositions according to the invention can also be for oral use, for example a toothpaste. In this case, the compositions may contain adjuvants and additives conventional for compositions for oral use, including surfactants, thickening agents, humectants, polishing agents such as silica, various active ingredients such as fluorides, in particular sodium fluoride, and optionally sweeteners such as saccharin sodium.

The material according to the present invention may be in the form of solution, dispersion, emulsion, paste, or powder, individually or as a premix or vehicled individually or as a premix in vectors such as macro-, micro-, or nanocapsules, macro-, micro- or , nanospheres, liposomes, oleosomes or chylomicrons, macro-, micro-, or nanoparticles or macro-, micro or nanosponges, micro- or nanoemulsions, or adsorbed on organic polymer powders, talcs, bentonites, spores or exines, and other inorganic or organic supports.

The material according to the present invention may be used in any form whatsoever, in a form bound to or incorporated in or absorbed in or adsorbed on macro-, micro-, and nanoparticles, or macro-, micro-, and nano-capsules, for the treatment of textiles, natural or synthetic fibers, wools, and any materials that may be used for clothing or underwear for day or night, handkerchiefs or cloths, intended to come into contact with the skin, to exert their cosmetic effect *via* this skin/textile contact and to permit continuous topical delivery.

### Cosmetic treatment method

The present invention also concerns a topical treatment method to improve the appearance and the general condition of the skin, the mucous membranes, and the appendages, comprising the topical application of an effective amount of a composition according to the invention as recited above.

The invention therefore relates to a topical cosmetic use of a material of the invention to improve the appearance and general condition of the skin and mucous membranes and skin appendages, including the fight against aging and/or skin imperfections of the skin and mucous membranes and skin appendages.

"Topical use" means an application that is intended to act where it is applied: skin, mucosa, skin appendages. A topical application can particularly be cosmetic, pharmaceutical or cosmeceutical.

The topical use of the invention can specifically target the chronological aging, hormonal aging, actinic aging (photodamage) or aging due to environmental effects.

One of the preferred modes of topical cosmetic use of the material according to the invention, or of a composition according to the invention, is intended, as a preventive or curative improvement of skin tone, of the radiance, of the firmness of the skin, for reducing wrinkles and fine lines, skin dryness, the imbalance of scalp, hair loss and brittle nails.

Owing to its properties, the material of the invention is also particularly suitable for the care and/or treatment of sensitive skin, sensitized skin, and/or reactive skin.

Improvements in the appearance and general condition of the skin, of mucous membranes, and of appendages can be obtained by topical application of these compositions on a regular basis such as daily.

The practitioner will appreciate the topical treatment that will include a composition containing the material of plant origin, this treatment can be achieved for example by applying topically to the composition described in the present invention, according to a method usually used to apply such a composition. The topical composition is preferably applied once daily for a period of at least a week, but it can be applied during periods of 2, 4, 8 or 12 weeks. The topical composition is preferably applied to the face and neck, but can be applied to any part of skin requiring an aesthetic improvement, where the composition remains on the skin area to be treated, and preferably is not removed or flushed from the skin.

It is also to be understood that, as used herein, the terms treating and treatment include and encompass the reduction, improvement, progress, relief, and/or elimination of dermatological effects, including aging. The compositions of the present invention and the methods are suitable for use to treat skin conditions of the skin in many areas of the skin, including without limitation, the face, forehead, lips, neck, neckline, arms, hands, body, legs, knees, feet, chest, back, buttocks, and others.

One of the major advantages of the present invention resides in the ability whenever necessary or desirable to be able to apply local selective "gentle" treatments through this topical, non-invasive method of application. In the case of anti-wrinkle use for example it may be applied very locally using a syringe or micro-canula.

It is also possible, however, to consider a composition containing the material of plant origin according to the invention intended to be injected subcutaneously.

According to other specific features, the cosmetic treatment method according to the invention can be combined with one or more other treatment methods targeting the skin such as lumino-therapy, heat or aromatherapy treatments.

According to the invention, devices with several compartments or kits may be proposed to apply the method described above which may include for example and non-restrictively, a first compartment containing a composition comprising the material or the extract of the invention, and in a second compartment a composition containing another active ingredient and/or excipient, the compositions contained in the said first and second compartments in this case being considered to be a combination composition for simultaneous, separate or stepwise use in time, particularly in one of the treatment methods recited above.

For a cosmeceutical or pharmaceutical application, composition according to the invention is an adapted form that can be ingested.

The invention also provides a composition comprising a material according to the invention for a therapeutical treatment of degraded skin, mucous membranes and skin appendages.

Other applications are possible, such as whitening, moisturizing, anti-wrinkle, firming, slimming, for increasing the volume of dermis and/or epidermis, anti-acne, anti-inflammatory, etc.

In particular in cosmetics, applications can be offered in particular in product lines for moisturizing, make up removal, cleansing, anti-aging, antioxidant, protective, restorative (hands, feet, lips), contours (face, eyes, neck, lips), the makeup-care of the skin and the appendages, including eyelashes, lip products, sunscreens, sculpting, plumping, volumizing (e.g. on the hands, chest, breasts), hair products, nail care etc..

### Additional ingredients

The CTFA International cosmetic ingredient dictionary & handbook (13th Ed. 2010) (published by the Cosmetic, Toiletry, and Fragrance Association, Inc., Washington, D.C.) describes a non-limited wide variety of cosmetic and pharmaceutical ingredients conventionally used in the skin care industry that can be used as additional ingredients/compounds in the compositions for the present invention. Examples of these ingredient classes include, but are not limited to: healing agents, skin anti-aging agents, anti-wrinkle agents, anti-atrophy agents, skin moisturizing agents, skin smoothing agents, antibacterial agents, anti-parasitic agents, antifungal agents, fungicidal agents, fungistatic agents, bactericidal agents, bacteriostatic agents, antimicrobial agents, anti-inflammatory agents, anti-pruriginous agents, anesthetic agents, antiviral agents, keratolytic agents, free radicals scavengers, anti-seborrhea agents, antidandruff agents, the agents modulating differentiation, proliferation or pigmentation of the skin and agents accelerating penetration, desquamating agents, melanin synthesis stimulating or inhibiting agents, whitening, depigmenting or lightening agents, pro-pigmenting agents, self-tanning agents, NO-synthase inhibiting agents, antioxidants, free radical scavengers and/or agents against atmospheric pollution, reactive carbonyl species scavengers, anti-glycation agents, tightening agents, agents stimulating the synthesis of dermal or epidermal macromolecules and/or capable of inhibiting or preventing their degradation, such as for example collagen synthesis stimulating agents, elastin synthesis stimulating agents, decorin synthesis stimulating agents, laminin synthesis stimulating agents, defensin synthesis stimulating agents, chaperone synthesis stimulating agents, aquaporin synthesis stimulation agents, hyaluronic acid synthesis stimulating agents, fibronectin synthesis stimulating agents, sirtuin synthesis-stimulating agents, agents stimulating the synthesis of lipids and components of the stratum corneum (ceramides, fatty acids, etc.), collagen degradation inhibiting agents elastin degradation inhibiting agents, agents stimulating fibroblast proliferation, agents stimulating keratinocyte proliferation, adipocyte proliferation stimulating agents, melanocyte proliferation stimulating agents, keratinocyte differentiation stimulating agents, adipocyte differentiation stimulating agents, acetylcholinesterase inhibiting agents, glycosaminoglycan synthesis stimulating agents, DNA repair agents, DNA protecting agents, anti-itching agents, agents for the treatment and/or care of sensitive skin, firming agents, anti-stretch mark agents, astringent agents, sebum production regulating agents, dermo-relaxing agents, healing adjuvant agents, re-epithelialization stimulating agents, re-epithelialization co-adjuvant agents, cytokine growth factors, calming agents, anti-inflammatory agents, agents acting on capillary circulation and/or microcirculation, angiogenesis stimulating agents, vascular permeability inhibiting agents, agents acting on cell metabolism, agents able to improve dermal-epidermal junction, agents inducing hair growth, hair growth inhibiting or retardant agents, muscle relaxants agents, antipollution and/or anti-free radical agents, lipolysis stimulating agents, slimming agents, anti-cellulite agents, agents acting on the microcirculation, agents acting on the energy metabolism of the cells, cleaning agents, hair conditioning agents, hair styling agents, hair growth promoters, sunscreen agents, total sunscreen agents, make-up agents, detergents, pharmaceutical products, emulsifiers, emollients, organic solvents, antiseptic agents, deodorant actives, physiologically acceptable carriers, surfactants, abrasives, absorbents, aesthetic components such as fragrances, pigments, dyes, colorants, natural colorants, essential oils, touch agents, cosmetic astringents, anti-acne agents, anti-coagulation agents, antifoaming agents, antioxidants, binders, biological additives, enzymes, enzymatic inhibitors, enzyme-inducing agents, coenzymes, chelating agents, plant extracts, plant derivatives, essential oils, marine extracts, agents obtained from a bio-fermentation or a biotechnological process, mineral salts, cell extracts, sunscreens (organic or mineral photoprotective agents active against ultraviolet A and/or B rays), ceramides, peptides, buffers, volumizing agents, chelating agents, chemical additives, colorants, cosmetic biocides, denaturants, medical astringents, external analgesics, film formers, such as polymers, for exacerbing film-forming properties and substantivity of the composition, quaternary derivatives, substantivity increasing agents, opacifying agents, pH adjusters and regulators (e.g. triethanolamine), propellants, reducing agents, sequestrants, decoloring and/or lightening agents, skin-conditioning agents (e.g., humectants, including miscellaneous and occlusive), moisture retaining agents, alphahydroxyacids, betahydroxyacids, moisturizers, epidermal hydrolytic enzymes, healing and/or calming agents, skin treating agents, anti-wrinkle agents, agents that reduce or treat bags under the eyes, exfoliating agents, thickeners, softening agents, gelling polymers, vitamins and their derivatives, wetting agents, peeling agents, soothing agents, curative agents of the skin, lignans, preservatives (i.e. phenoxyethanol and parabens), anti UV, cytotoxic agents, anti-neoplastics, viscosity modifiers, non-volatile solvents, pearling agents, anti-perspirant agents, depilatories, vaccine, perfumed water, skin restructuring agent (i.e. Siegesbeckia orientalis extract), excipients, charges, minerals, anti-mycobacterial agents, anti-allergenic agents, H1 or H2 antihistaminics, anti-irritants, agents stimulating the immune system, agents inhibiting the immune system, insect repellents, lubricants, pigments or dyes, hypopigmentation agents, preservatives, light stabilizers, and mixtures thereof, as long as they are physically and chemically compatible with the other ingredients of the composition and especially with the active ingredients of the present invention. Also the nature of these additional ingredients should not unacceptably alter the benefits of the active ingredients of the invention. These additional ingredients can be synthetic or natural such as plants extracts, or come from a bio-fermentation process. Additional examples can be found in the CTFA Cosmetic Ingredient Handbook.

Such additional active ingredient/compound can be selected from the group consisting of: sugar amines, glucosamine, D-glucosamine, N-acetyl glucosamine, N-acetyl-D-glucosamine, mannosamine, N-acetyl mannosamine, galactosamine, N-acetyl galactosamine, B3 vitamin and its derivatives, niacinamide, sodium dehydroacetate, dehydroacetic acid and its salts, phytosterols, salicylic acid compounds, hexamidines, dialkanoyl hydroxyproline compounds, soy extracts and derivatives, equol, isoflavones, flavonoids, phytantriol, farnesol, geraniol, bisabolol, peptides and their derivatives, di-, tri-, tetra-, penta-, and hexapeptides and their derivatives, KTTKS (SEQ ID NO: 4), Pal-KTTKS (SEQ ID NO: 2), carnosine, N-acyl amino acid compounds, retinoids, retinyl propionate, retinol, retinyl palmitate, retinyl acetate, retinal, retinoic acid, water-soluble vitamins, ascorbates, C vitamin, ascorbyl glucoside, ascorbyl palmitate, magnesium ascorbyl phosphate, sodium ascorbyl phosphate, vitamin B and their salts and derivatives, B1 vitamin, B2 vitamin, B6 vitamin, B12 vitamin, provitamins and their salts and derivatives, K vitamin and derivatives, pantothenic acid and its derivatives, pantothenyl ethyl ether, panthenol and derivatives, dexpanthenol, biotin, amino acids and their salts and derivatives, water soluble amino acids, asparagine, alanine, indole, glutamic acid, water insoluble vitamins, A vitamin, E vitamin, F vitamin, D vitamin and mono-,di-, and tri-terpenoids compounds, beta-ionol, cedrol, and their derivatives, water insoluble amino acids, tyrosine, tryptamine, particulate materials, butylated hydroxytoluene, butylated hydroxyanisole, allantoin, tocopherol nicotinate, tocopherol, tocopherol esters, palmitoyl-Gly-His-Lys (Pal-GHK), phytosterol, hydroxy acids, glycolic acid, lactic acid, lactobionic acid, keto acids, pyruvic acid, phytic acid, lysophosphatidic acid, stilbenes, cinnamates, resveratrol, kinetin, zeatin, dimethylaminoethanol, natural peptides, soy peptides, salts of sugar acids, Mn gluconate, Zn gluconate, piroctone olamine, 3,4,4'-trichlorocarbanilide, triclocarban, Zn pyrithione, hydroquinone, kojic acid, ascorbic acid, magnesium ascorbyl phosphate, ascorbyl glucoside, pyridoxine, aloe vera, terpene alcohols, allantoin, bisabolol, dipotassium glycyrrhizinate, glycerol acid, sorbitol acid, pentaerythritol acid, pyrrolidone acid and salts, dihydroxyacetone, erythrulose, glyceraldehyde, tartaraldehyde, clove oil, menthol, camphor, eucalyptus oil, eugenol, menthyl lactate, witch hazel distillate, eicosene and vinyl pyrrolidone copolymer, iodopropyl butylcarbamate, a polysaccharide, an essential fatty acid, a salicylate, glycyrrhetinic acid, carotenoids, ceramides and pseudo-ceramides, a lipid complex, oils in general of natural origin such shea butter, apricot oil, onagre oil, prune oil, palm oil, monoi oil, hydroquinone, HEPES, procysteine, O-octanoyl-6-D-maltose, the disodium salt of methylglycinediacetic acid, steroids such as diosgenin and derivatives of DHEA, DHEA or dehydroepiandrosterone and/or a precursor or chemical or biological derivative thereof, N-ethyloxycarbonyl-4-para-aminophenol, blueberries extracts, phytohormones, extracts of the yeast Saccharomyces cerevisiae, extracts of algae, extracts of soyabean, lupin, maize and/or peas, alverine and its salts, in particular alverine citrate, extract of butcher's broom and of horse chestnut, octadecenedioic acid, analogs and derivatives, and mixtures thereof, a metalloproteinase inhibitor.

Further skin care and hair care active ingredients that are particularly useful combined with the composition can be found in SEDERMA commercial literature and on the website www.sederma.fr. The following commercial actives can also be mentioned, as examples: betain, glycerol, Actimoist Bio 2™ (Active organics), AquaCacteen™ (Mibelle AG Cosmetics), Aquaphyline™ (Silab), AquaregulK™ (Solabia), Carciline™ (Greentech), Codiavelane™ (Biotech Marine), Dermaflux™ (Arch Chemicals, Inc), Hydra'Flow™ (Sochibo), Hydromoist L™ (Symrise), RenovHyal™ (Soliance), Seamoss™ (Biotech Marine), Essenskin™ (Sederma), Moist 24™ (Sederma), Argireline™ (trade name of the acetyl hexapeptide-3 of Lipotec), spilanthol or an extract of Acmella oleracea known under the name Gatuline Expression™, an extract of Boswellia serrata known under the name Boswellin™, Deepaline PVB™ (Seppic), Syn-AKE™ (Pentapharm), Ameliox™, Bioxilift™ (Silab), Juvinity™ (Sederma), Revidrate™ (Sederma), Chronodyn™ (Sederma), O.D.A.white™ (Sederma), Idealift™ (Sederma), Resistem™ (Sederma), Prodizia™ (Sederma), Widelash™ (Sederma) or mixtures thereof.

Among other plant extracts which can be combined with the composition of the invention, there may more particularly be mentioned extracts of Ivy, in particular English Ivy (*Hedera Helix*), of *Bupleurum chinensis,* of *Bupleurum Falcatum*, of arnica (*Arnica Montana L*), of rosemary (*Rosmarinus officinalis N*), of marigold (*Calendula officinalis*), of sage (*Salvia officinalis L*), of ginseng (*Panax ginseng*), of ginko biloba, of St.-John's-Wort (*Hyperycum Perforatum*), of butcher's-broom (*Ruscus aculeatus L*), of European meadowsweet (*Filipendula ulmaria L*), of big- flowered Jarva tea (*Orthosiphon Stamincus Benth*), of algae (*Fucus Vesiculosus*), of birch (*Betula alba*), of green tea, of cola nuts (*Cola Nipida*), of horse-chestnut, of bamboo, of *Centella asiatica*, of heather, of fucus, of willow, of mouse-ear, of escine, of cangzhu, of *chrysanthellum indium*, of the plants of the *Armeniacea* genus, *Atractylodis Platicodon, Sinnomenum*, *Pharbitidis, Flemingia,* of *Coleus* such as *C*. *Forskohlii, C. blumei, C. esquirolii*, *C. scutellaroides*, *C. xanthantus* and *C. Barbatus*, such as the extract of root of *Coleus barbatus*, extracts of *Ballote*, of *Guioa*, of *Davallia*, of *Terminalia*, of *Barringtonia*, of *Trema*, of *antirobia*, *cecropia*, *argania*, *dioscoreae* such as *Dioscorea opposita* or Mexican, extracts of *Ammi visnaga*, of *Siegesbeckia,* in particular *Siegesbeckia orientalis*, vegetable extracts of the family of *Ericaceae,* in particular bilberry extracts (*Vaccinium angustifollium*) or *Arctostaphylos uva ursi*, *aloe vera*, plant containing sterols (e.g., phytosterol), Manjistha (extracted from plants of the genus *Rubia*, particularly *Rubia Cordifolia*), and Guggal (extracted from plants of the genus *Commiphora*, particularly *Commiphora Mukul*), kola extract, chamomile, red clover extract, Piper methysticum extract (Kava Kava™ from SEDERMA), *Bacopa monieri* extract (Bacocalmine™ from SEDERMA) and sea whip extract, extracts of *Glycyrrhiza glabra*, of mulberry, of *melaleuca* (tea tree), of *Larrea divaricata*, of *Rabdosia rubescens,* of *Euglena gracilis,* of *Fibraurea recisa Hirudinea*, of *Chaparral Sorghum*, of sun flower extract, of *Enantia chlorantha,* of Mitracarpe of *Spermacocea* genus, of *Buchu barosma,* of *Lawsonia inermis L.,* of *Adiantium Capillus-Veneris L.,* of *Chelidonium majus*, of *Luffa cylindrica*, of Japanese Mandarin (*Citrus reticulata Blanco* var. *unshiu*), of *Camelia sinensis*, of *Imperata cylindrica*, of *Glaucium Flavum,* of *Cupressus Sempervirens*, of *Polygonatum multiflorum*, of *loveyly hemsleya*, of *Sambucus Nigra*, of *Phaseolus lunatus*, of *Centaurium*, of *Macrocystis Pyrifera*, of *Turnera Diffuse*, of *Anemarrhena asphodeloides,* of *Portulaca pilosa,* of *Humulus lupulus*, of *Coffea Arabica*, of *Ilex Paraguariensis*, of *Zingimber Zerumbet Smith*, *of Globularia Cordifolia, of Albizzia Julibrissin*, *or of Ulva Lactuca.*

The compositions of the present invention may include peptides, including, without limitation, the di-, tri-, tetra-, penta-and hexapeptides and their derivatives. According to a particular embodiment, the concentration of the additional peptide, in the composition, ranges from 1x10⁻⁷% and 20%, preferably from 1x10⁻⁶% and 10%, preferably between 1x10⁻⁵% and 5%, by weight.

According to the present invention, the term "peptide" refers to peptides containing 10 amino acids or less, their derivatives, isomers and complexes with other species such as a metal ion (e.g. copper, zinc, manganese, magnesium, and others). The term "peptides" refers to both natural peptides and synthetic peptides. It also refers to compositions that contain peptides which are found in nature, and/or are commercially available.

Suitable dipeptides for use herein include but are not limited to carnosine (beta-AH), YR, VW, NF, DF, KT, KC, CK, KP, KK or TT. Suitable tripeptides for use herein include, but are not limited to RKR, HGG, GHK, GKH, GGH, GHG, KFK, KPK, KMOK, KMO₂K or KAvaK. Suitable tetrapeptides for use herein include but are not limited to RSRK (SEQ ID NO: 5), GQPR (SEQ ID NO: 6) or KTFK (SEQ ID NO: 7). Suitable pentapeptides include, but are not limited to KTTKS (SEQ ID NO: 4). Suitable hexapeptides include but are not limited to GKTTKS (SEQ ID NO: 8), VGVAPG (SEQ ID NO: 9).

Other suitable peptides for use herein include, but are not limited to lipophilic derivatives of peptides, preferably palmitoyl derivatives, and metal complexes as aforementioned (e.g. copper complex of the tripeptide HGG). Preferred dipeptide derivatives include N-Palmitoyl-beta-Ala-His, N-Acetyl-Tyr-Arg-hexadecylester (Calmosensine™, Idealift™ from Sederma). Preferred tripeptide derivatives include N-Palmitoyl-Gly-Lys-His, and Pal-Gly-His-Ly, (Pal-GKH and Pal-GHK from Sederma), the copper derivative of HGG (Lamin™ from Sigma), Lipospondin (N-Elaidoyl-KFK) and its analogs of conservative substitution, N-Acetyl-RKR-NH2 (Peptide CK+), N-Biot-GHK (from Sederma), Pal-KMO2K (Sederma) and derivatives thereof. Suitable tetrapeptide derivatives for use according to the present invention include, but are not limited to, N-palmitoyl-GQPR (SEQ ID NO: 3) (from Sederma), suitable pentapeptide derivatives for use herein include, but are not limited to, N-Palmitoyl-KTTKS (SEQ ID NO: 2) (available as Matrixyl™ from Sederma), N-Palmitoyl-Tyr-Gly-Gly-Phe-X (SEQ ID NO: 10) with X Met or Leu or mixtures thereof. Suitable hexapeptide derivatives for use herein include, but are not limited to, N-Palmitoyl-VGVAPG (SEQ ID NO: 1) and derivatives thereof. The mixture of Pal-GHK and Pal-GQPR (SEQ ID NO: 3) (Matrixyl™ 3000, Sederma) can also be mentioned.

The preferred compositions commercially available containing a tripeptide or a derivative include Biopeptide-CL™, Maxilip™, Biobustyl™, Procapil™ and Matrixyl™synthe'6™ of Sederma. The compositions commercially available preferred sources of tetrapeptides include Rigin™, Eyeliss™, Matrixyl™ Reloaded and Matrixyl 3000™ which contain between 50 and 500 ppm of Palmitoyl-GQPR (SEQ ID NO: 3) and carrier, proposed by Sederma.

The following marketed peptides can be mentioned as well as additional active ingredients: Vialox™, Syn-ake™ or Syn-Coll™ (Pentapharm), Hydroxyprolisilane CN™ (Exsymol), Argireline™, Leuphasyl™, Aldenine™, Trylgen™, Eyeseryl™, Serilesine™ or Decorinyl™ (Lipotec), Collaxyl™ or Quintescine™ (Vincience), BONT-L-Peptide™ (Infinitec Activos), Cytokinol™LS (Laboratoires Serobiologiques/Cognis), Kollaren™, IP2000™ or Meliprene™ (Institut Européen de Biologie Cellulaire), Neutrazen™ (Innovations), ECM-Protect™ (Atrium Innovations), Timp-Peptide™ or ECM Moduline™ (Infinitec Activos).

### Techniques of in vitro plant culture

Among the today existing technics in the field of *in vitro* plant culture, the followings can be used according to the invention:
Culture of undifferentiated cells: This method involves first the creation of highly proliferative cell lines in agar medium. These lines are then grown in liquid medium in order to substantially increase the biomass. At the end of the growth cycle and environmental conditions to be defined and optimized (searching for the appropriate elicitation parameters), the cell biomass will synthesize the molecules of interest. The culture is then stopped and extracted at the optimum time to obtain an extract, said to be of plant origin, containing a maximum amount of molecules of interest. Existing cell lines already available can also be used initially.
Culture of tissue or organ: This type of culture can concern the root part ("root culture") or the aerial part ("shoot culture"), or somatic embryos ("somatic embryo"). In this type of method, there are cultures that have been transformed by genomics bacteria *Agrobacterium rhizogenes* (root) or *Agrobacterium tumefaciens* (stems, leaves, flowers, etc.). Roots or aerial parts cultures thus transformed have a high growth rate and are genetically very stable. They are used to synthesize the molecules of interest after optimizing the elicitation parameters. These molecules are then extracted by conventional means.
*In vitro* micropropagation through vegetative multiplication, in particular somatic embryogenesis: The first step for this technique is to select a parent plant with optimal characteristics (in terms of growth and production of metabolites of interest). In embryogenesis, the next step is the induction of callus from explants from the parent plant. These undifferentiated cells can give rise to numerous somatic embryos that it will be possible to multiply and extend through successive cycles of culture. From the lines of the most proliferative and productive, seedlings are regenerated in a suitable medium and extracted in at the appropriate time for optimal production of metabolites of interest. The second step is the induction of stems with leaves from explants of the parent plant on a suitable culture medium thanks to its hormonal composition. An amplification phase of leafy stem is followed by a rooting phase to induce roots. It is then possible to regenerate plantlets in large quantities, to grow them in a suitable medium and extract the molecules of interest.

The material of plant origin of the present invention is preferentially obtained by the first technique of *in vitro* plant culture, from a line of undifferentiated plant cells. This technique is described in more detail below.

The first step of obtaining a cell culture involves the creation of a cell line.

Said cell line may be an existing line or a line created in a preliminary stage, with said step being performed separately in time.

According to the invention, the step of creating the cell line comprises the steps 1) induction of callus (clusters of undifferentiated cells), 2) selection of the best callus line and 3) transfer to cell suspensions and optimization of the growth parameters and production of molecules of interest (e.g. elicitation).

More particularly:
1) The induction of callus (clusters of undifferentiated cells) can be done with all parts of the plant, including leaf, fruit, root, bud, seed, stem, branch, and meristematic tissues, in particular cambium. Preferably, according to the invention, the undifferentiated cell culture is achieved from the aerial parts (leaves and stems) of the plant.
2) The selection of the best callus or cells lines, before a transfer in liquid medium, is performed according in particular to the following criteria: high proliferative capacity, tender and friable texture, uniform color, good dispersion in liquid medium and long-term stability of these parameters. The culture medium is optimized.
   The creation of a cell line may include a Phase 3) Optimization on the selected calli, in liquid medium, of the growth parameters, and production of molecules of interest or secondary metabolites (e.g. by elicitation). This step goes through the choice of the most appropriate media, in a first step to ensure rapid growth of the biomass and in a second time to allow maximum efficiency of secondary metabolites synthesis by cells at the end of exponential phase (stop of the multiplication). This last action corresponds to the culture elicitation and can be achieved in different ways among those available to the skilled person. These include in particular: the addition to the cultivation of microbial fractions, of stress molecules known to direct cells to their secondary metabolism; the application to the culture of a change in temperature or pH, or of an osmotic stress; the use of an impoverishment of the environment (such a medium deficient in sucrose and macronutrients); the adding to the culture of adsorbent resins, which in addition to eliciting the compounds of interest can trap them.

The creation of the cell line being formed, obtaining cell suspensions is presented below:
- A step of pre-cultures, for increasing the biomass, comprises the completion of pre-cultures of increasing volumes, each pre-culture of smaller size for generating cellular biomass in sufficient quantities to inoculate the pre-culture of a larger size.
- The subsequent step of cultures in bioreactor, comprises a phase of cell proliferation in a base medium. An optional phase of elicitation can be achieved in a bioreactor inoculated with the biomass produced in the pre-culture step. A medium of elicitation of secondary metabolites is added to the bioreactor. Culture is stopped when the desired level of secondary metabolites has been achieved.
- The recovery step (or harvesting step) may include an extraction stage of this biomass by any physiologically acceptable solvent, or any mixture of these solvents. The extraction can be done by different known methods that can be combined: heat, maceration, decoction, infusion, pressure, leaching, ultrasonic, microwave, by lysing the cells by any chemical or physical appropriate method. Phase separation can be achieved by filtration or centrifugation. Alternatively, it is possible to extract the biomass with a supercritical fluid or subcritical. Alternatively also, it is possible to achieve a purification using an adsorbent resin, chromatographic methods or liquid-liquid partition. According to a preferred embodiment, the cells are lysed. After several filtrations, a sterile extract is obtained.

The following examples describe and illustrate certain aspects of the invention. They should not be perceived as limiting the disclosure, because they mainly provide useful information for its understanding and implementation.

### A) Example of obtaining a materiel of plant origin according to the invention by undifferentiated cell culture of Oxydendrum arboreum

### 1) Creation of a cell line

*Oxydendrum Arboreum* plants were obtained by seed germination in sterile conditions on an agar medium. After about one month, aerial parts (leaves and stems) were removed from the seedlings and cut into explants.

### a) Step of induction of undifferentiated cells or callus

These explants (pieces of leaves and stems) are placed on the surface of a nutrient agar, containing an assimilable carbon source, a solution of micro and macro-elements and an adapted combination of hormones and vitamins. This nutrient medium, thanks to its composition, will trigger the production of clusters of undifferentiated cells called callus at the site of healing of the leaf or stem.

Composition of the solid nutrient medium used: solution of macro-elements and micro-elements, saccharose, agar plant, plant hormones and vitamins. These media may be those of Murashig and shoog or Shenk and Hildebrandt or Gamborg's.

This medium will be thereafter referred to as the "basic medium".

This induction step lasts 1 to 4 months. Culture is made at 25 ° C in the dark or photoperiod of 16h of light and 8h of darkness.

### b) Stabilization and selection step of the cultures in agar medium.

Callus stabilization is obtained after successive transplantations, every 4 weeks, on fresh medium. Stable and homogeneous cultures are thus obtained thanks to their phenotypic characteristics (color, friability, proliferation).

The selection is to choose the best callus cultures for the transfer in liquid medium, among the tens initiated earlier. The selected lines are characterized by high growth, a soft and crumbly texture, uniform color, good dispersion in liquid medium and stability of these parameters during the subcultures.

This step lasts from 6 months to 1 year.

### c) transfer in liquid medium and optimization of the growth parameters

The selected lines are transferred in liquid medium in order to optimize their growth parameters.

Cell growth and appreciation of the different phases (latence, exponential, stationary) are assessed by regular measurement of the percentage volume occupied by the culture cells or "packed cell volume" (PCV).

Different suspensions from the callus are placed on an orbital shaker, agitated at 110 rpm in the dark at between 23°C and 27°C (in Erlenmeyer flasks each containing 100 ml of medium and 10 grams of callus homogeneous in texture, color and growth). Several media and hormone combinations are tested and compared in order to optimize the growth curve. The optimization of the initial density of the inoculum is also seeked for reducing the lag phase and thus the global duration of culture.

In the end, the medium used is the basic medium for a liquid medium.

This transfer phase in liquid medium and optimization of the parameters of growth and production can last from 6 months to 1 year.

After this step, a line giving the best results was selected.

### 2) Extract obtaining

### a) Production of pre-cultures in bioreactors

Successive pre-cultures of the cell line selected of 1-liter, then 3, 10, 30 liter, and then 90 liter and finally 270 liter are realized. Each is seeded at 10% PCV and then grown to 30% of PCV. The cell biomass thus produced is used to inoculate the larger size culture. This stage can last from 1 to 3 months.

### b) Recovery of the extract

The aim of this subsequent step is to produce an aqueous and clarified extract.

A cell lysis is achieved. The cells wall is destroyed and cell fragments are removed. A cascade filtration is then carried out, the first in-depth, followed by sterile filtration, to obtain the aqueous extract of the invention. This step is intended to stabilize the extract.

### B) Example of formulation of a composition for implementing the invention which is an "active ingredient", a raw material for the cosmetics industry

The active ingredient is a composition according to the invention containing the material of plant origin of *Oxydendrum arboreum* of the invention dissolved in a matrix forming a physiologically acceptable medium. This active ingredient is in particular intended for the cosmetics industry for the preparation of cosmetics, such as creams, gels, etc. (see galenic examples given below).

The aqueous extract obtained in Example A) above may preferably be mixed at 20 to 80% with any hydrophilic matrix as a gel, an aqueous buffer, glycerin or other short-chain polyol physiologically acceptable.

For example, and for the achievement of the studies and galenic presented thereafter, an extract up to 33% in glycerin was preferentially used.

Of course, a pure material of plant origin, without excipient, can also be used as the active ingredient.

### C) In vitro Studies

The material of plant origin according to the invention of *Oxydendrum arboreum* presents a number of remarkable effects which are presented below.

An extract of *Oxydendrum arboreum* cell culture prepared according to Example B) above, was tested *in vitro* and showed various activities, concerning several areas of the skin physiology.

### 1. Anti-glycation

Protein glycation is a form of biochemical degradation of the skin. This phenomenon increases with age and contributes to the continuing deterioration of cells. In the dermis, it primarily affects the collagen fibers, contributing to the loss of elasticity of this tissue. These reactions between sugars and proteins will also generate free radicals, which are themselves the cause of damage to the dermal extracellular matrix. So, the stiffening of the dermis and a decrease in turnover of macromolecules are resulting from glycation.

Therefore an active ingredient having an anti-glycation property is particularly prized for its effects on skin elasticity, skin radiance, and anti-aging in general.

A simple test reproduces *in vitro* a stress to which are regularly subjected skin proteins: their functional alteration by a non-enzymatic glycation phenomenon.

The study model used is a bovine serum albumin (BSA), which is contacted with a physiological reducing sugar, the fructose, giving a spontaneous and slow reaction between the two molecules that can be accelerated by temperature.

In this test, the BSA and fructose are incubated in the presence or absence of the test products at 50°C (temperature accelerates the process) for 7 days. The glycation end products have a natural fluorescence that can be quantified using a fluorimeter (λex=360nm and λem=460nm).

0.03% Aminoguanidine is used as a positive control of glycation inhibition.

**Table 1 : Non-enzymatic glycation inhibition in the presence of the extract of Oxydendrum arboreum cell culture compared to the control. (n=4)**

| Control | Concentration | % Change vs control |
|---|---|---|
| Negative Control | - | 23145 +/- 264 |
| | | Reference |
| Extract of *Oxydendrum arboreum* cell culture | 100ppm | -20% ; p<0.01 |
| | 300ppm | -62% ; p<0.01 |

The extract of *Oxydendrum arboreum* cell culture inhibits non-enzymatic glycation in a dose-dependent and significant way.

### 2. Anti-radical/anti-oxidant Test

It is now recognized that it is essentially the accumulation of reactive oxygen species (ROS) which is the origin of biochemical disorders themselves responsible for aging. Whether generated by UV (*via* a complex cascade of reactions), by the different pollutants that interact with skin chronically or by natural aging, those free radicals will cause skin aging punctually and for a short period of skin inflammation, and throughout the body and a on a longer period. In so-called normal conditions, in a youthful skin, healthy and relatively protected from external attacks, there is a balance between the production of reactive oxygen species (ROS) in the body and antioxidant defenses responsible for neutralizing them. These defense systems are carried out either by enzymes (SOD, catalase, glutathione peroxidase) or by conventional antioxidants (vitamin C, vitamin E, glutathione, carotene ...). When an imbalance occurs between production and neutralization, then there is overabundance of reactive oxygen species (ROS) causing oxidative stress. This will result in damage to DNA, proteins and cellular lipids, which will undergo such oxidations, causing alterations in their structure and function. Among the many targets of ROS, are especially polyunsaturated fatty acids, which will destabilize the peroxidation of cell membranes. The spread of self-catalytic primary oxidations can lead to a complete disorganization of the membrane.

### 2.1. DPPH anti-radical test

The DPPH (1,1-diphenyl-2-picryl hydrazyl) is a stable free radical which is widely used for the detection of free radical scavengers. This molecule, by losing its radical character is converted to DPPH2 (1,1-diphenyl-2-picryl hydrazine). This conversion is accompanied by discoloration (purple → yellow) which can be monitored over time by spectrophotometry at 517nm. The absorption at this wavelength comes from its extra electron. This absorption decreases on a stoichiometric way depending on the number of electrons captured.

Control with no antiradical activity keeps a constant DO. 0.003% of caffeic acid is used as a positive control for anti-radical activity.

**Table 2: percentage of protection in the presence of an extract of Oxydendrum arboreum cell culture compared to the control. (n=6)**

| | Concentration | **DPPH** |
|---|---|---|
| Extract of *Oxydendrum arboreum* cell culture | 10ppm | |
| | 30ppm | +4% |
| | | p<0.01 |
| | 100ppm | +11% |
| | | p<0.01 |
| | 200ppm | +45% |
| | | p<0.01 |

The extract of *Oxydendrum arboreum* cell culture presents a potential antiradical very marked and significant (p <0.01). The anti-radical protection is dose dependent from 10 to 200ppm.

### 2.2.Anti-singlet-oxygen test

Molecular oxygen (O₂) plays a vital role as an acceptor of the respiratory chain terminal electrons. However, this metabolism and others, as well as external factors, such as UV radiations, lead to the occurrence of certain derivatives particularly reactive (singlet oxygen, hydroxyl radical, superoxide anion), which will cause oxidative damage to lipids, proteins and nucleic acids, even in healthy bodies. Singlet oxygen is one of these very energetic and highly reactive forms of oxygen. It is possible to produce experimentally *in vitro* singlet oxygen with dyes and UVA photons or visible light. The test presented thereafter uses the Rose Bengal, a dye and visible light.

The purpose of this test is to evaluate the degradation of uric acid by singlet oxygen formed from the Rose Bengal, under the action of visible light. The visualization of the production of singlet oxygen is obtained by following the destruction of uric acid at 292 nm. In the presence of a compound capable of neutralizing singlet oxygen, this destruction will be slowed.

The caffeic acid at 0.002% is used as a positive control of an anti-singlet-oxygen activity.

**Table 3 : percentage of protection in the presence of the extract of Oxydendrum arboreum cell culture compared with control. (n=6)**

| | Concentration | **Anti O2 singlet** |
|---|---|---|
| *Oxydendrum arboreum* cell culture extract | 10ppm | +5% |
| | | nsd |
| | 30ppm | +11% |
| | | p<0.01 |
| | 100ppm | +23% |
| | | p<0.01 |
| | 200ppm | - |

| | | |
|---|---|---|
| *nsd* = non-significant difference. | | |

The extract of *Oxydendrum arboreum* cell culture presents a potential antioxidant very marked and significant (p <0.01). The antioxidant protection is dose dependent from 10 to 200ppm.

### 2.3.Anti-lipoperoxidation UVA test

Lipid peroxidation is a phenomenon with many steps: initiation, propagation (autocatalytic) and termination. The phenomenon is initiated by a subtraction of H⁺ to polyunsaturated fatty acids. The termination generates small molecules (aldehydes, hydroxyalcenane ...) toxic and mutagenic. The test is to have a model of lipid membranes as liposomes that are exposed to an UVA oxidant radiation (Lipoperoxidation UVA induced). The appearance of an oxidation is observed by evaluating the amount of conjugated dienes formed. Their reduction by use of an antioxidant is sought.

**Table 4 : percentage of protection in the presence of the extract of Oxydendrum arboreum cell culture compared with control. (n=6)**

| | Concentration | **Lipoperoxidation** |
|---|---|---|
| *Oxydendrum arboreum* cell culture extract | 10ppm | +68% |
| | | p<0.01 |
| | 30ppm | +74% |
| | | p<0.01 |
| | 100ppm | +76% |
| | | p<0.01 |
| | 200ppm | - |

| | | |
|---|---|---|
| *Oxydendrum arboreum* cell culture extract presents a potential antioxidant very marked and significant (p <0.01). | | |

### 3. Effects on extracellular matrix

Matrix metalloproteinases or MMPs are enzymes that degrade the extracellular matrix of connective tissues, including skin. There are many types (about thirty). Particularly interstitial collagenase or MMP1 is synthesized by fibroblasts or polynuclears and degrades the fibrillar collagens I, II and III; and gelatinases (MMP-2 or gelatinase A (72 kDa), MMP-9 or gelatinase B (92 kDa)) degrade collagen type IV or any form of denatured collagen.

### 3.1. Inhibition of the synthesis of Matrix Metalloproteinase-1 (MMP1)

Confluent fibroblasts are exposed to the products to be tested for 24 hours. Cell layers were then irradiated (UVA 3J/cm²) and the cells undergo a new contact of 24 hours with the products. The MMP1 were determined by ELISA.

**Table 5 : percentage of MMP1 synthesis observed in the presence of the extract of Oxydendrum arboreum cell culture compared with control. (n=4)**

| | Concentration | **% inhibition of MMP1 synthesis / Control** |
|---|---|---|
| *Oxydendrum arboreum* cell culture extract | 10ppm | -10% |
| | | *dns* |
| | 30ppm | -31% |
| | | *p<0.01* |

| | | |
|---|---|---|
| *Oxydendrum arboreum* cell culture extract inhibits on a dose-dependent and significant way the MMP1 synthesis by fibroblasts. The product has not been toxic to NHF. | | |

### 3.2.Inhibition of the activity of Matrix Metalloproteinases 2 and 9 (MMP2 and MMP9)

A peptide substrate (gelatin model, a degraded collagen) conjugated to fluorescein is used. The natural fluorescence of this fluorophore is quenched (off) when bound to gelatin. The collagenases used (Metalloproteinase-2 or MMP2, or Metalloproteinase-9 or MMP9) will, during the cleavage of the substrate, allow the intrinsic fluorescence *via* the release of labeled fragments. Fluorescence, no longer quenched, is measurable in the test medium by a fluorometer (λex = 485 nm, λem = 520 nm). The change in fluorescence is proportional to the proteolytic activity of collagenase.

**Table 6 : percentage of MMP2 & MMP9 activity in the presence of Oxydendrum arboreum cell culture extract compared with control. (n=4)**

| | Concentration | MMP2 Activity mU +/- sd % Change / control | MMP9 Activity mU +/- sd % Change / control |
|---|---|---|---|
| Negative Control | - | 255 +/- 7 | 405 +/- 23 |
| | | Reference | Reference |
| *Oxydendrum arboreum* cell culture extract | 100ppm | 126 +/- 6 | 262 +/- 20 |
| | | **-51 ; p<0.01** | **-35 ; p<0.01** |
| | 200ppm | 63 +/- 2 | 193 +/- 9 |
| | | **-75 ; p<0.01** | **-52 ; p<0.01** |
| | 300ppm | 38 +/- 1 | 142 +/- 9 |
| | | **-85 ; p<0.01** | **-65 ; p<0.01** |

| | | | |
|---|---|---|---|
| sd = standard deviation | | | |

The extract of *Oxydendrum arboreum* cell culture inhibits on a dose-dependent and significant way the MMP2 and MMP9 activities.

### 3.3. Increased synthesis of collagen I by dermal human fibroblasts

Normal human fibroblasts (NHF) are cultivated in plate MW24 for 24 hours. The cells are in contact or not of test products at various concentrations for 7 days. The synthesis of collagen I is then quantified by immuno-labeling fixed on the layer.

**Table 7: increase of collagen I on NHF in the presence of the extract of Oxydendrum arboretum cell culture compared with control. (n=4 wells/ 20 photos)**

| | Concentration | Collagen I Synthesis % change / control |
|---|---|---|
| *Oxydendrum arboreum* cell culture extract | 10ppm | +47 ; p<0.01 |
| | 30ppm | +117 ; p<0.01 |
| Positive control (Vitamin C) | => | +236% ; p<0.01 |

The extract of *Oxydendrum arboreum* cell culture increases on a dose-dependent and significant way the collagen I synthesis on NHF.

### 4. Anti-inflammatory effect

Normal human fibroblasts (NHF) are cultivated and brought into contact with the test products at various concentrations for 24 h and then the layers are irradiated using a non-cytotoxic dose of UVB (70mJ/cm²). The irradiated cells were subsequently handed over in contact with new products for 24 hours. The amount of IL-8 synthesized was measured in culture supernatants by ELISA.

**Table 8: inhibition of IL8 synthesis post UVB in the presence of the extract of Oxydendrum arboreum cell culture compared with control. (n=3)**

| | Concentration | **IL8** (% Change / control) |
|---|---|---|
| Positive control | => | -54 |
| | | p<0.01 |
| *Oxydendrum arboreum* cell culture extract | **50ppm** | -15 |
| | | p<0.05 |
| | **100ppm** | -37 |
| | | p<0.01 |

The extract of *Oxydendrum arboreum* cell culture inhibits moderately but significantly and on a dose-dependent way the IL8 synthesis after a stress of UVB type.

### 5. Effect on pigmentation

The cosmetics industry is in search of compounds with depigmenting properties (bleaching, pigmentation and skin lightening; elimination or reduction of freckles, age spots etc.). *In vitro,* it is possible to demonstrate such an effect by determining the melanin synthesized and tyrosinase activity, on melanocyte cultures having been or not in contact with the compounds to be evaluated.

Human melanocytes were seeded in 24-well plates. After 24 h of attachment, the cells are in contact with the test products. After 5 days of culture, the layers are rinsed, the total melanins are extracted in sodium hydroxide and assayed spectrophotometrically at 490 nm. For tyrosinase, an identical culture protocol is followed and then the enzyme is extracted from the cells and DOPA oxidase activity is measured spectrophotometrically at 37°C with a solution of L-DOPA to 0.2%. The data are normalized using a viability assay.

**Table 9: inhibition of melanogenese in the presence of the extract of Oxydendrum arboreum cell culture compared with control. (n=5)**

| | Concentration | **Melanin** (% Change / Control) | **Tyrosinase** (% Change / Control) |
|---|---|---|---|
| Arbutin (positive control) | => | -56 | -48 |
| | | p<0.01 | p<0.01 |
| *Oxydendrum arboreum* cell culture extract | **50ppm** | -10 | -14 |
| | | p<0.05 | p<0.05 |
| | **100ppm** | -21 | -25 |
| | | p<0.01 | p<0.01 |
| | **200ppm** | -30 | -33 |
| | | p<0.01 | p<0.01 |

The extract of *Oxydendrum arboreum* cell culture inhibits moderately but significantly and on a dose-dependent way the melanin synthesis and the tyrosinase activity of human melanocyte.

### D) Galenic

### Active ingredient according to the invention:

Formula comprising the material of plant origin, and in particular the *Oxydendrum arboreum* cell culture extract according to the invention (as disclosed in above example B).

Different formulas are described below with or without additional cosmetic active ingredients, the latter coming as appropriate to support and/or in addition to the activity of the active ingredient of the invention. These ingredients can be of any class according to their(s) function(s), site of application (body, face, neck, chest, hands, etc.), the desired final effect and the target consumer, such as anti-aging, anti-wrinkle, moisturizing, circles and/or bags under the eyes, firming, brightening, anti-glycation, anti-spots, slimming, soothing, muscle-relaxant, anti-redness, anti-stretch marks, etc.

### Example 1: Day cream form for face

| **Ingredients** | **INCI names** | **% by weight** |
|---|---|---|
| **Phase A** | | |
| H₂O | Water | Qsp100 |
| Ultrez 10 | Carbomer | 0.25 |

| **Phase B** | | |
|---|---|---|
| Butvlen glycol | Butylene glycol | 2.00 |
| Phenoxyethanol | Phenoxyethanol | qs |

| **Phase C** | | |
|---|---|---|
| Brij S2/Volpo S2 | Steareth-2 | 0.40 |
| Brij S10/Volpo S 10 | Steareth-10 | 1.20 |
| Crodafos CES | Cetearyl alcohol & Dicetyl phosphate & Ceteth 10 phosphate | 4.00 |
| Crodacol CS 90 | Cetearyl Alcohol | 1.00 |
| Laurocapram | Azone | 2.50 |
| DC 345 | Cyclohexasiloxane & Cyclopentasiloxane | 2.00 |
| Crodamol OSU | Ethylhexyl succinate | 7.00 |

| **Phase D** | | |
|---|---|---|
| Potassium sorbate | Potassium Sorbate | 0.10 |

| **Phase E** | | |
|---|---|---|
| H₂O | Water | 3.00 |
| NaOH 30 % | Sodium Hydroxide | 0.40 |

| **Phase F** | | |
|---|---|---|
| Active ingredient of the invention | | 3.00 |

**Procedure:** weigh Phase A and let swell without stirring for 30 min. Heat Phase A to 75°C in a water bath. Weigh and mix Phase B. Weigh Phase C and heat to 75°C in a water bath. Add Phase B into Phase A. Mix well. Under stirring add Phase C into Phase (A+B). Homogenize well. Add Phase D, extemporaneously. Add Phase E, homogenize well. Add Phase F, mix thoroughly.

### Example 2: Gel form for the face

| **Ingredients** | **INCI names** | **% by weight** |
|---|---|---|
| **Phase A** | | |
| H₂O | Water | Qsp100 |
| Cetyl hydroxyethylcellulose | Cetyl hydroxyethylcellulose | 0.30 |

| **Phase B** | | |
|---|---|---|
| Ultrez 10 | Carbomer | 0.40 |
| H₂O | Water | 20.00 |

| **Phase C** | | |
|---|---|---|
| Glycerin | Glycerin | 3.00 |
| Panstat | Ethyl & Methyl & Propyl parabens | 0.30 |

| **Phase D** | | |
|---|---|---|
| Marcol 82 | Mineral oil | 4.00 |
| Crillet 1 | Polysorbate 20 | 1.00 |
| Crodamol AB | C12-15 Alkyl Benzoate | 2.00 |
| Pemulen TR2 | C10-30 Alkyl Acrylate cross polymer | 0.30 |

| **Phase E** | | |
|---|---|---|
| Potassium sorbate | Potassium Sorbate | 0.10 |

| **Phase F** | | |
|---|---|---|
| H₂O | Water | 5.00 |
| NaOH 10N | Sodium Hydroxide | 0.50 |

| **Phase G** | | |
|---|---|---|
| Active ingredient of the invention | | 2.00 |

**Procedure:** Disperse Phase A under stirring. Sprinkle Ultrez 10 in water, let stand 30 minutes. Heat Phase C until it is completely dissolved. Mix Phase A with Phase B. Add Phase C in Phase (B+A). Add Phase D under in phase (A+B+C). Add Phase E. Neutralize with Phase F. Add Phase G and mix.

**Examples of additional ingredients** that can be added to the gel type formula in one of the phases or extemporaneously according to their hydrophobic or hydrophilic physical property at a certain % depending on their concentration and the desired effect:
RIGIN^{™}: active marketed by SEDERMA (WO2000/433417) improving the elasticity and firmness of the skin, increasing hydration and smoothing the skin. 3% by weight of this ingredient may for example be added to the formulation in phase G.
RENOVAGE^{™}: global anti-aging active ingredient marketed by SEDERMA (WO2006/020646). 3% by weight of this ingredient may for example be added to the formulation in Phase D.
LUMISKIN^{™}: active ingredient marketed by SEDERMA (WO2004/024695), which lightens the complexion. 3% by weight of this ingredient may for example be added to the formulation in Phase D.
SUBLISKIN^{™}: active ingredient marketed by SEDERMA (WO2009/055663) that moisturizes and smooths the skin while allowing it to resist to external aggressions. 3% by weight of the ingredient may for example be added to the formulation in phase G.
MATRIXYL 3000^{™}: anti-wrinkle peptide-based active ingredient marketed by SEDERMA (WO2005/048968) which helps to repair skin damages caused by aging. 3% by weight of this ingredient may for example be added to the formulation in phase G.

### Example 3: Night cream form for face

| **Ingredients** | **INCI names** | **% by weight** |
|---|---|---|
| **Phase A** | | |
| H₂O | Water | qsp100 |
| Ultrez 10 | Carbomer | 0.40 |

| **Phase B** | | |
|---|---|---|
| Glycerin | Glycerin | 3.00 |
| Panstat | Ethyl & Methyl & Propyl parabens | 0.30 |

| **Phase C** | | |
|---|---|---|
| Polawax GP 200 | Cetearyl Alcohol & polysorbate 20 | 1.00 |
| Crodacol CS 90 | Cetearyl Alcohol | 1.00 |
| Crodamol STS | PPG-3 Benzyl Ether Myristate | 1.00 |
| DC 200 5 cps | Dimethicone | 2.50 |
| Crodamol TN | Isotridecyl Isononanoate | 5.00 |

| **Phase D** | | |
|---|---|---|
| Potassium sorbate | Potassium sorbate | 0.10 |

| **Phase E** | | |
|---|---|---|
| NaOH 30% | Sodium hydroxide | 0.40 |
| H₂O | Water | 4.00 |

| **Phase F** | | |
|---|---|---|
| Active ingredient of the invention | | 2.00 |

| **Phase G** | | |
|---|---|---|
| Perfume | Fragrance | 0.10 |

**Procedure:** Weigh Phase A and let swell for 30 minutes. Then heat Phase A in a water bath at 75°C; heat Phase B until dissolved. Add Phase B to Phase A. Heat Phase C in a water bath at 75°C. Under stirring, add Phase C in Phase (A+B). Add Phase D, homogenize well. Neutralize with Phase E around 55°C. Add Phase F, then Phase G, homogenize well.

**Examples of additional ingredients** that can be added to the cream type formula in one of the phases or extemporaneously according to their hydrophobic or hydrophilic physical property at a certain % depending on their concentration and the desired effect:
DERMAXYL^{™}: anti-aging active ingredient marketed by SEDERMA (WO2004/101609) that smoothes wrinkles and repairs the skin barrier. 3% by weight of this ingredient can be added just before use, for example in phase (A+B+C).
Niacinamide (Vitamin B3), Retinol, resveratrol, DHEA: anti-aging ingredients, including anti-wrinkle. 0.5% by weight of retinol, resveratrol or DHEA may for example be added extemporaneously to the phase (A+B+C). 10 wt% of Niacinamide 10% in water for example, can be added to the phase G.
Hexamidine: anti-bacterial active that can be added to the G phase of the formulation to 0.5% by mass. CHRONODYN^{™}: active ingredient marketed by SEDERMA (WO2006/075311) which tones and firms the skin, erases visible signs of fatigue. 3% of this ingredient may for example be added to the phase G.
VENUCEANE^{™}: active ingredient marketed by SEDERMA (WO2002/066668) that prevents visible signs of photo-ageing (spots, wrinkles, dryness, etc.), protects cell structures from damages caused by UV and strengthens skin integrity. 3% of this ingredient may for example be added to the phase G.

### Example 4: Cream form for the body

| **Ingredients** | **INCI names** | % **by weight** |
|---|---|---|
| **Phase A** | | |
| H₂O | Water | qsp 100 |
| Ultrez 10 | Carbomer | 0.40 |

| **Phase B** | | |
|---|---|---|
| Glycerin | Glycerin | 3.00 |
| Panstat | Ethyl & Methyl & Propyl parabens | 0.30 |

| **Phase C** | | |
|---|---|---|
| Crill 3 | Sorbitan Stearate | 2.00 |
| Marcol 82 | Mineral oil | 4.00 |
| Cromollient DP3A | PPG 3 Myristyl Ether Adipate | 1.00 |
| Cithrol GMS AS | Glyceryl stearate & PEG 100 stearate | 3.00 |

| **Phase D** | | |
|---|---|---|
| Potassium sorbate | Potassium sorbate | 0.10 |

| **Phase E** | | |
|---|---|---|
| NaOH 30% | Sodium hydroxide | 0.40 |
| H₂O | Water | 4.00 |

| **Phase F** | | |
|---|---|---|
| Active ingredient of the invention | | 3.00 |

| **Phase G** | | |
|---|---|---|
| Fragrance | Fragrance | 0.10 |

**Procedure**: Weigh Phase A and let swell for 30 minutes. Heat Phase A in a water bath at 75°C; heat Phase B until dissolved. Add Phase B to Phase A. Heat Phase C in a water bath at 75°C. Under stirring, add Phase C in Phase (A+B). Add Phase D, homogenize well. Neutralize with Phase E around 55°C. Add Phase F, then Phase G, homogenize well.

**Examples of additional ingredients** that can be added to the cream type formula in one of the phases or extemporaneously according to their hydrophobic or hydrophilic physical property at a certain % depending on their concentration and the desired effect:
JUVINITY^{™}: active marketed by SEDERMA reducing signs of aging on the face and neckline, smoothing wrinkles, densifying and restructuring the dermis. 2% of this ingredient may for example be added extemporaneously to Phase (A+B+C).
Tocopherol or vitamin E*:* actives having anti-radical and antioxidant properties.
O.D.A. white^{™}: active marketed by SEDERMA (WO1994/07837) which lightens the skin by reducing the synthesis of melanin. 1% of that ingredient may for example be added extemporaneously to Phase (A+B+C).
Tocopherol (vitamin E) or α-lipoic acid (ALA): active with anti-oxidant and anti-radical properties. 0.5% by weight can be added for example to Phase (A+B+C).
Bio-Bustyl^{™}: active marketed by SEDERMA based on peptide and a bacterial filtrate having a global action on firmness and tone of the bust. 3% of this ingredient can be added for example to Phase G.

### Example 5: Serum form

| **Ingredients** | **INCI names** | **% by weight** |
|---|---|---|
| **Phase A** | | |
| Optasens G 40 | Carbomer | 0.25 |
| H₂O | Water | Qsp100 |

| **Phase B** | | |
|---|---|---|
| Butylene Glycol | Butylene Glycol | 3.00 |
| Phenoxyethanol | Phenoxyethanol | 0.20 |

| **Phase C** | | |
|---|---|---|
| Crillet 1 | Polysorbate 20 | 0.50 |
| DC 245 | Cyclopentasiloxane | 1.00 |
| Crodamol CAP | Cetearvl Ethylhexanoate | 2.00 |
| Crodamol STS | PPG-3 Benzyl Ether Myristate | 0.50 |
| Pemulen TR2 | Acrylates/C 10-30 Alkyl Acrylates cross | 0.20 |
| | polymer | |

| **Phase D** | | |
|---|---|---|
| Potassium Sorbate | Potassium Sorbate | 0.10 |

| **Phase E** | | |
|---|---|---|
| H₂O | Water | 4.00 |
| NaOH 30 % | Sodium Hydroxiide | 0.45 |

| **Phase F** | | |
|---|---|---|
| Active ingredient of the invention | | 2.00 |

| **Phase G** | | |
|---|---|---|
| Fragrance | Fragrance | 0.10 |

**Procedure**: Phase A: Sprinkle carbomer in water, let swell 15 minutes. Mix Phase B. Pour Phase B in Phase A and homogenize. Weigh Phase C, mix and add in Phase (A+B), under stirring. Let swell 1 hour. Extemporaneously add Phase D in the previous phase under stirring. Neutralize with Phase E. Put under stirring. Then add Phase F. Allow mixing at least one hour under stirring and then add Phase G. Mix well.

**Examples of additional ingredients** that can be added to the serum type formula in one of the phases or extemporaneously according to their hydrophobic or hydrophilic physical property at a certain % depending on their concentration and the desired effect:
LUMISHERE™: active ingredient marketed by SEDERMA (WO2004/024695). It is the combination of diacétylboldine (DAB) encapsulated in polymethylmethacrylate microcapsules and titanium dioxide modified with manganese (TiO₂Mn). The TiO₂Mn gives the skin a unifying, mattifying and luminous effect and DAB provides a physiological Phase lightening effect. 4% of this ingredient may for example be added to the Phase F of the formulation.
REVIDRATE^{™}: active marketed by SEDERMA that in particular improves the cohesion of the epidermis and its hydration. 2% of this ingredient may for example be added to Phase C of the formulation.
EVERMAT^{™}: active marketed by SEDERMA (WO2007/029187), which decreases the secretion of sebum and thus participates in the treatment of oily skin. 4% of this ingredient may for example be added to Phase F of the formulation.
HALOXYL^{™}: active marketed by SEDERMA (WO2005/102266), which improves the eye contour by reducing dark circles. 3% of this ingredient may for example be added to Phase F of the formulation.

### Example 6: Lotion form

| **Ingredients** | **INCI names** | **% by weight** |
|---|---|---|
| **Phase A** | | |
| H₂O | Water | Qsp100 |

| **Phase B** | | |
|---|---|---|
| Butylene Glycol | Butylene Glycol | 5.00 |
| Phenoxyethanol | Phenoxyethanol | 0.20 |

| **Phase C** | | |
|---|---|---|
| Crillet 1 | Polysorbate 20 | 2.00 |
| Crodamol STS | PPG-3 Benzyl Ether Myristate | 0.10 |

| **Phase D** | | |
|---|---|---|
| Potassium Sorbate | Potassium Sorbate | 0.10 |

| **Phase E** | | |
|---|---|---|
| Active ingredient of the invention | | 2.00 |

| **Phase F** | | |
|---|---|---|
| Fragrance | Fragrance | 0.10 |

**Procedure**: Weigh Phase A. Weigh Phase B and mix. Add Phase B into Phase A under stirring for 30 minutes. Weigh Phase C, mix until obtaining a homogenized mixture. Add Phase C into phase A+B while stirring. Add Phase D in the previous Phase. Add Phase E still under stirring; homogenise well. Weigh Phase F, mix and add to the previous Phase, mix thoroughly.

**Examples of additional ingredients** that can be added to the lotion type formula in one of the phases or extemporaneously according to their hydrophobic or hydrophilic physical property at a certain % depending on their concentration and the desired effect:
EYELISS^{™}: an active sold by SEDERMA (WO2003/068141), which helps prevent and fight against the appearance of bags under the eyes. 3% of this ingredient may for example be added to Phase E of the formula.
Ac-Net^{™}: an active sold by SEDERMA (WO2003/02828692) offering a complete treatment of oily and acne-prone skins. 3% of this ingredient may for example be added to Phase E of the formula.
EVERMAT^{™}: mentioned above. 4% of this ingredient can be for example added to Phase E of the formula.
HYDRERGY^{™}: active marketed by SEDERMA (WO2003/02828692 long-term moisturizer and stimulates the ATP synthesis. 3% of this ingredient may for example be added to Phase E of the formula.

### Example 7 : Day cream

| **Ingredients** | **INCI names** | **% by weight** |
|---|---|---|
| **Phase A** | | |
| Water | | 81.70 |
| Ultrez 10 | Carbomer | 0.20 |

| **Phase B** | | |
|---|---|---|
| Butylene glycol | Butylene glycol | 2.00 |
| Phenoxyethanol | Phenoxyethanol | 1.30 |

| **Phase C** | | |
|---|---|---|
| Cithrol GMS A/S NA | Glyceryl stearate & PEG 100 stearate | 1.00 |
| Crodamol GTCC | Caprylic /Capric Triglycerides | 4.00 |

| **Phase D** | | |
|---|---|---|
| Pemulen TR2 | Acrylates/ C 10-30 Alkyl Acrylates cross polymer | 0.20 |
| Cromollient STS | PPG-3 Benzyl Ether Myristate | 1.00 |
| DC 200 | Dimethicone | 1.00 |

| **Phase E** | | |
|---|---|---|
| Sorbate | Sorbate | 0.10 |

| **Phase F** | | |
|---|---|---|
| NaOH 30% | Sodium hydroxyde | 0.40 |
| H₂O | | 4.00 |

| **Phase G** | | |
|---|---|---|
| Active ingredient of the invention | | 3.00 |

| **Phase H** | | |
|---|---|---|
| fragrance | Fragrance | 0.10 |

**Procedure**: Phase A: pour Ultrez 10 in water and let swell for 30 min. Weigh Phase B and let melt at 60°C. Heat Phase A in a water bath at 75°C. Weigh Phase C and heat to 75°C in a water bath. Under stirring Staro v=500 rpm, add Phase C into Phase A. Extemporaneously, add Phase B and Phase D into (A+C), homogenize well, add then Phase E, homogenize well. Cool to 45°C and add Phase F. At about 35°C, add Phase G, homogenize well, then add Phase H, homogenize well.

### Exemple 8: Fluid for the body

| **Ingredients** | **INCI names** | **% by weight** |
|---|---|---|
| **Phase A** | | |
| H₂O | Water | 5.00 |
| Ultrez 10 | Carbomer | 0.30 |

| **Phase B** | | |
|---|---|---|
| H₂O | Water | 74.30 |
| Natrosol 250 | Hydroxyethyl cellulose | 0.20 |

| **Phase C** | | |
|---|---|---|
| Butylene Glycol | Butylene Glycol | 3.00 |
| Phenoxyethanol | Phenoxyethanol | 1.30 |

| **Phase D** | | |
|---|---|---|
| Crodamol AB | C12-15 Alkyl Benzoate | 2.00 |
| Crodamol GTCC | Caprylic/Capric Triglyceride | 3.00 |
| Crillet 1 | Polysorbate 20 | 1.00 |
| Crodamol STS | PPG-3 Benzyl Ether Myristate | 1.00 |
| Pemulen TR2 | C 10-30 Alkyl Acrylate Cross Polymer | 0.20 |

| **Phase E** | | |
|---|---|---|
| Sorbate | Potassium Sorbate | 0.10 |

| **Phase F** | | |
|---|---|---|
| NaOH 30 % | Sodium Hydroxyde | 0.50 |
| H₂O | Water | 5.00 |

| **Phase G** | | |
|---|---|---|
| Active ingredient of the invention | | 3.00 |

| **Phase H** | | |
|---|---|---|
| Fragrance | Fragrance | 0.10 |

**Procedure**: Phase A: Pour Ultrez 10 in water and let swell for 30 min. Under stirring propeller 300rpm pour Phase B and let swell for 1h. Add Phase A into Phase B, under propeller stirring 300rpm, homogenize well. Weigh and stir Phase C, weigh Phase D and mix. Add Phase C into Phase A+B. Add Phase D into Phase A+B+C. Homogenize well. Add Phase E, then Phase F and G, finally Phase H. pH = 6.2

### Example 9 : Night cream:

| **Ingredients** | **INCI names** | **% by weight** |
|---|---|---|
| **Phase A** | | |
| H₂O | | 5.00 |
| Ultrez 10 | Carbomer | 0.30 |

| **Phase B** | | |
|---|---|---|
| H₂O | | 80.10 |
| Natrosol 250 | Hydroxyethyl cellulose | 0.40 |

| **Phase C** | | |
|---|---|---|
| Butylene Glycol | Butylene Glycol | 3.00 |
| Phenoxyethanol | Phenoxyethanol | 1.30 |

| **Phase D** | | |
|---|---|---|
| Crodamol STS | PPG-3 Benzyl Ether Myristate | 1.00 |
| Pemulen TR2 | C 10-30 Alkyl Acrylate Cross Polymer | 0.20 |

| **Phase E** | | |
|---|---|---|
| Sorbate | Potassium Sorbate | 0.10 |

| **Phase F** | | |
|---|---|---|
| NaOH 30 % | Sodium Hydroxyde | 0.50 |
| H₂O | | 5.00 |

| **Phase G** | | |
|---|---|---|
| Active ingredient of the invention | | 3.00 |

| **Phase H** | | |
|---|---|---|
| Fragrance | Fragrance | 0.10 |

**Procedure**: Phase A: Pour Ultrez 10 in water and let swell for 30 min. Under stirring propeller 300rpm pour Phase B and let swell for 1h. Add Phase A into Phase B, under propeller stirring 300rpm, homogenize well. Weigh and stir Phase C, weigh Phase D and mix. Add Phase C into Phase A+B under blade stirring 300rpm. Add Phase D into Phase A+B+C. Homogenize well. Add Phase E, then Phase F and G, finally Phase H. Homogenize well.

### Example 10 : Anti Stretch-mark Cream

| **Ingredients** | **INCI name** | **% by weight** |
|---|---|---|
| **Phase A** | | |
| Water deionised | Water (Aqua) | qsp 100% |
| Ultrez 10 | Carbomer | 0.40 |

| **Phase B** | | |
|---|---|---|
| Glycerin | | 5.00 |
| Phenova | Phenoxyethanol (and) Mixed Parabens | 0.80 |

| **Phase C** | | |
|---|---|---|
| Crodamol OP | Ethylhexyl Palmitate | 4.00 |
| Crodacol CS90 | Cetearyl alcoholCroda | 0.50 |
| Crodamol ML | Myristyl Lactate | 0.30 |
| Crillet 1 | Polysorbate 20 | 1.00 |

| **Phase D** | | |
|---|---|---|
| Pemulen TR2 | Acrylates / C10-30 Alkyl Acrylate Crosspolymer | 0.20 |
| DC 345 | Cyclomethicone | 2.00 |

| **Phase E** | | |
|---|---|---|
| Potassium Sorbate | Potassium Sorbate | 0.10 |

| **Phase F** | | |
|---|---|---|
| NaOH 38% | Sodium Hydroxide | 0.60 |
| Water deionised | Water (Aqua) | 6.00 |

| **Phase G** | | |
|---|---|---|
| Active ingredient of the invention | | 2.00 |

| **Phase H** | | |
|---|---|---|
| UNISLIM® | Ilex Paraguariensis (Leaf) Extract - Aqua (Water) - Butylene Glycol - Coffea Arabica (Coffee Seed) Bean Extract - PEG-60 Almond Glycerides - Glycerin - Cetyl Hydroxyethylcellulose | 3.00 |
| MATRIXYL® 3000 | Glycerin (and) Butylene Glycol (and) Aqua (Water) (and) Carbomer (and) Polysorbate-20 (and) Palmitoyl Oligopeptide (and) Palmitoyl Tetrapeptide-3 | 3.00 |
| DARUTOSIDE | *Siegesbeckia Orientalis* Extract | 3.00 |

| | | |
|---|---|---|
| DARUTOSIDE is a molecule sold by SEDERMA for the treatment of stretch marks. | | |

**Procedure:** This emulsion is prepared in the following way: Phase A: disperse Ultrez 10 in water and let it swell for 20 minutes. Mix Phase B and heat to 60°C until dissolution. Add Phase B to Phase A while stirring. Heat Phase (A+B). Weigh Phase C and heat to 75°C. Add Phase C to Phase (A+B) under stirring. Homogenise carefully, then add Phase D. Add Phase E extemporaneously. Then at about 50°C neutralise with Phase F. Add Phases G and H at about 35°C; adjust pH to ~6.30 with NaOH.

### Example 11: Hair lotion:

| **Ingredients** | **INCI names** | **% by weight** |
|---|---|---|
| **Phase A** | | |
| Incroquat CTC 30 | Cetrimonium chloride | 1.00 |
| Citric acid | Citric Acid | 0.22 |
| Citrate Trisodique | Citrate trisodique | 1.20 |
| Sorbate | Potassium Sorbate | 0.10 |
| H₂O | | Qsp |

| **Phase B** | | |
|---|---|---|
| Nipagine | Methyl paraben | 0.20 |
| Procetyl AWS | PPG 5 Ceteth 20 | 2.00 |

| **Phase C** | | |
|---|---|---|
| Active ingredient of the invention | | 2.00 |

| **Phase D** | | |
|---|---|---|
| Crillet 1 | Polysorbate 20 | 1.00 |
| Fragrance | Fragrance | 0.10 |

### Example 13: Moisturizing makeup

| **Ingredients** | **INCI name** | **% by weitght** |
|---|---|---|
| **Phase A** | | |
| Water deionised | Water (Aqua) | qsp 100% |
| KOH 10% | Potassium hydroxide | 1.30 |
| Crillet 4 NF | Polysorbate 80 | 0.10 |

| **Phase B** | | |
|---|---|---|
| Titanium dioxide | | 6.00 |
| Talc | | 3.05 |
| Yellow iron oxide | | 1.80 |
| Red iron oxide | | 1.00 |
| Black iron oxide | | 0.15 |

| **Phase C** | | |
|---|---|---|
| Propylene glycol | | 4.00 |
| Veegum Regular | Magnesium Aluminum Silicate | 1.00 |

| **Phase D** | | |
|---|---|---|
| Propylene glycol | | 2.00 |
| Cellulose gum | Sodium Carboxymethylcellulose | 0.12 |

| **Phase E** | | |
|---|---|---|
| Cromollient DP3-A | Di-PPG-3 Myristyl Ether Adipate | 12.00 |
| Crodamol ISNP | Isostearyl Neopentanoate | 4.00 |
| Crodafos CS 20 | Cetearyl Alcohol (and) Ceteth-20 Phosphate (and) Dicetyl Phosphate | 3.00 |
| Volpo S-10 | Steareth-10 | 2.00 |
| Crodacol C-70 | Cetvl alcohol | 0.62 |
| Volpo S-2 | Steareth-2 | 0.50 |

| **Phase F** | | |
|---|---|---|
| Moist 24® | Imperata Cylindrica extract (and) water (and) glycerine (and) PEG8 (and) carbomer | 3.00 |

| **Phase G** | | |
|---|---|---|
| Active ingredient of the invention | | 5.00 |

| **Phase H** | | |
|---|---|---|
| Germaben II | Propylene glycol (and) Diazolidinyl Urea (and) Methylparaben (and) Propylparaben | 1.00 |

**Procedure**: Phase A: disperse Ultrez 10 in water and let it swell for 20 minutes. Premix the pigments, then add Phase B to Phase A until the pigments are dispersed: begin heating. Heat phase C separately, mix well. Pour Phase C in Phase (A+B) while stirring. Prepare phase D separately, mix well. Pour Phase D in Phase (A+B+C) while stirring; mix thoroughly. Prepare Phase E separately, mix well. Pour Phase E in Phase (A+B+C+D) with stirring; around 45°C add Phase F and G and H. Adjust the pH to 7.5, homogenize.

Moist 24® is an extract of Cylindrica imperata root extract offrered by par Sederma (WO 01/62218)

### Example 14: Lip balm

| **Ingredients** | **INCI names** | **% by weight** |
|---|---|---|
| **Phase A** | | |
| Water deionised | | qsp 100% |
| Sorbate | Potassium Sorbate | 0.10 |
| Magnesium Sulfate | Magnesium Sulfate | 0.70 |

| **Phase B** | | |
|---|---|---|
| Abil EM 90 | Cetyl Dimethicone Copolyol | 3.00 |
| Methyl Paraben | Methyl Paraben | 1.00 |
| Svncrowax HRC | Tribehenin | 0.30 |
| Crodamol STS | PPG-3 Benzyl Ether Myristate | 2.00 |
| Mineral Oil | Mineral Oil | 19.00 |

| **Phase C** | | |
|---|---|---|
| Active ingredient of the invention | | 1.00 |

| **Part D** | | |
|---|---|---|
| Fragrance | Fragrance | 0.10 |

**Procedure**: Heat Phase A at 85°C. Mix Phase B and heat to 85°C. Mix. Slowly add Phase A to Phase B with stirring (Staro s = 3000, then s = 1200). Add Phase C preheated to 80°C, homogenize. Add Phase D to 35°C. Pour.

### Example 15: Hair Spray protector

| **Ingredients** | **INCI name** | **% by weight** |
|---|---|---|
| **Phase A** | | |
| Water deionised | Water (Aqua) | qsp 100% |
| Ethanol | | 10.00 |
| Crillet 1 | Polysorbate 20 | 0.40 |
| Incroquat CTC 30 | Cetrimonium Chloride | 1.00 |

| **Phase B** | | |
|---|---|---|
| HELIOGENOL™ | Butylene Glycol (and) Helianthus Annuus (Sunflower) Seed Extract | 5.00 |
| Preservatives | | qs |

| **Phase C** | | |
|---|---|---|
| Active ingredient of the invention | | 4.00 |

| **Phase D** | | |
|---|---|---|
| Water deionised | Water (Aqua) | 0.50 |
| NaOH | Sodium Hydroxide | 0.05 |

### Procedure: Weigh and mix Phase A with stirring propeller s = 300rpm; Add Phase B, mix, then the Phase C. Adjust the pH to ~ 5 to 5.5 with Phase D.

HELIOGENOL™ is an extract of sunflower meal proposed by Sederma (EP0512040).

## Claims

1. Material of plant origin which is a cell extract obtained by *in vitro* plant culture of *Oxydendrum arboreum.*

2. Material according to claim 1, which is obtained by undifferentiated plant cells culture.

3. Material according any of preceding claims, which is obtained from aerial parts, leaves and stems.

4. Material according any of preceding claims for use as a cosmetic active ingredient for improving the appearance and general condition of the skin, mucous membranes, and appendages.

5. Material according to preceding claim for use as a cosmetic active ingredient for fighting against aging and/or the imperfections of the skin, mucous membranes, and appendages.

6. Topical composition which contains, in a physiologically acceptable medium, a material according to any claim 1 to 5.

7. Composition according to claim 6, wherein the material is present in a sufficient quantity to have an anti-radical and anti-oxidant activity, an inhibition activity of glycation and of metalloproteinases (MMPs), an activity of increasing collagen, an inhibition activity of inflammation and melanogenesis.

8. Composition according to claims 6 and 7, which comprises at least one additional active ingredient.

9. Composition according to claim 8, wherein said additional active ingredient is chosen among whitening agents, anti-redness, UV sunscreens, moisturizing, humectant, exfoliating, anti-aging, anti-wrinkles and anti-fine lines, thinning, volumizing, improving the elastic properties, anti-acne, anti-inflammatory, anti-oxidant, anti-radical, anti-glycant, propigmenting or depigmenting, depilatories, anti-growth or promoting hair growth, peptides, vitamins agents.

10. Topical cosmetic use of a material of plant origin obtained from *Oxydendrum arboreum* or of a material according to any claims 1 to 3, for improving the appearance and general condition of the skin, mucous membranes, and appendages, and in particular for fighting against aging and/or the imperfections of the skin, mucous membranes, and appendages.

11. Use according to claim 10 wherein aging is chronological, hormonal or actinic (photodamage) aging, or caused by environmental stress.

12. Use according to any of claims 10 or 11, wherein the improvement of the appearance and general condition is the improvement of skin tone, of the complexion, of the firmness of the skin, a decrease of wrinkles and fine lines, of skin dryness, of the imbalance of scalp, of hair loss, of brittle nails.

13. Use according to any of claims 10 to 12, wherein the skin is a sensitive skin, a sensitized skin, or a reactive skin.

14. Composition comprising a material according any of claims 1 to 3, for a therapeutical treatment of degraded skin, mucous membranes and skin appendages.

## Patentansprüche

1. Material pflanzlichen Ursprungs, das ein Zellextrakt ist, den man durch eine *in vitro* Pflanzenkultur von *Oxydendrum arboreum* erhalten hat.

2. Material nach Anspruch 1, das man durch eine undifferenzierte Pflanzenzellenkultur erhalten hat.

3. Material nach einem der vorhergehenden Ansprüche, das man von oberirdischen Teilen, Blättern und Stielen erhalten hat.

4. Material nach einem der vorhergehenden Ansprüche zur Verwendung als kosmetischer Wirkstoff zur Verbesserung des Erscheinungsbildes und des allgemeinen Zustandes der Haut, der Schleimhäute und der Anhangsgebilde.

5. Material nach einem der vorhergehenden Ansprüche zur Verwendung als kosmetischer Wirkstoff zur Bekämpfung der Alterung und/oder von Unvollkommenheiten der Haut, der Schleimhäute und der Anhangsgebilde.

6. Topische Zusammensetzung, die in einem physiologisch annehmbaren Medium ein Material nach einem der Ansprüche 1 bis 5 enthält.

7. Zusammensetzung nach Anspruch 6, wobei das Material in einer ausreichenden Menge vorhanden ist, so dass es eine antiradikale und antioxidative Wirkung, eine hemmende Wirkung der Glykierung und Metalloproteinasen (MMP), eine Wirkung von zunehmendem Kollagen, eine hemmende Wirkung der Entzündung und Melanogenese hat.

8. Zusammensetzung nach Anspruch 6 und 7, die mindestens einen zusätzlichen Wirkstoff aufweist.

9. Zusammensetzung nach Anspruch 8, wobei der zusätzliche Wirkstoff ausgewählt ist aus Weißmachern, Anti-Redness, UV-Sonnenschutzmitteln, Feuchtigkeitscremes, Feuchthaltemitteln, Peeling, Anti-Aging, Antifalten creme und Mitteln gegen feine Linien, Ausdünnen, Volumengebung, Verbessern der elastischen Eigenschaften, Anti-Aknemitteln, Entzündungshemmern, Antioxidantien, Anti-Radikalen, Anti-Glykanen, Propigmentierung oder Depigmentierung, Enthaarungsmitteln, Anti-Wachstums- oder Haarwuchs fördernden Mitteln, Peptiden, Vitaminen.

10. Topische kosmetische Verwendung eines Materials pflanzlichen Ursprungs, erhalten aus *Oxydendrum arboreum* oder einem Material nach einem der Ansprüche 1 bis 3, zur Verbesserung des Erscheinungsbildes und des allgemeinen Zustands der Haut, der Schleimhäute und Anhangsgebilde, und insbesondere zur Bekämpfung des Alterns und/oder von Unvollkommenheiten der Haut, der Schleimhäute und Anhangsgebilde.

11. Verfahren nach Anspruch 10, wobei das Altern chronologisches, hormonelles oder aktinisches (Lichtschädigung) Altern oder verursacht durch Umweltbelastung ist.

12. Verwendung nach einem der Ansprüche 10 oder 11, wobei die Verbesserung des Erscheinungsbildes und des allgemeinen Zustandes die Verbesserung der Hautfarbe, des Teints, der Festigkeit der Haut, ein Rückgang von Falten und feinen Linien, der Hauttrockenheit, der Unausgeglichenheit der Kopfhaut, von Haarverlust, von brüchigen Nägeln ist.

13. Verwendung nach einem der Ansprüche 10 bis 12, wobei die Haut eine empfindliche Haut, eine sensibilisierte Haut oder eine reaktive Haut ist.

14. Zusammensetzung aufweisend ein Material nach einem der Ansprüche 1 bis 3 für eine therapeutische Behandlung von geschädigter Haut, Schleimhäuten und Hautanhangsgebilden.

## Revendications

1. Matériel d'origine végétale **caractérisé en ce qu'**il s'agit d'un extrait cellulaire obtenu par culture végétale *in vitro* d'*Oxydendron arboreum*.

2. Matériel selon la revendication 1, **caractérisé en ce qu'**il est obtenu par culture de cellules végétales indifférenciées.

3. Matériel selon l'une quelconque des revendications précédentes, **caractérisé en ce qu'**il est obtenu à partir des parties aériennes, de feuilles et de tiges.

4. Matériel selon l'une quelconques des revendications précédentes pour une utilisation comme ingrédient actif cosmétique pour améliorer l'apparence et l'état général de la peau des muqueuses et des phanères.

5. Matériel selon la revendication précédente pour une utilisation comme ingrédient actif cosmétique pour lutter contre le vieillissement et/ou les imperfections de la peau, des muqueuses ou des phanères.

6. Composition topique **caractérisée en ce qu'**elle contient, dans un milieu physiologiquement acceptable, un matériel selon l'une des revendications 1 à 5.

7. Composition selon la revendication 6, **caractérisée en ce que** le matériel est présent en quantité suffisante pour avoir une activité anti-radicalaire et anti-oxydante, une activité d'inhibition de la glycation et des métallo-protéinases (MMPs), une activité d'augmentation de la synthèse de collagène, et une activité d'inhibition de l'inflammation et de la mélanogénèse.

8. Composition selon les revendications 6 et 7, **caractérisée en ce qu'**elle contient au moins un ingrédient actif additionnel.

9. Composition selon la revendication 8, **caractérisée en ce que** ledit ingrédient actif additionnel est choisi parmi choisis parmi les actifs éclaircissants, anti-rougeurs, les filtres solaires UV, les actifs hydratants, humectants, exfoliants, anti-âges, anti-rides et ridules, amincissants, volumateurs, améliorant les propriétés élastiques, anti-acné, anti-inflammatoires, antioxydants, anti-radicalaires, anti-glycants, propigmentants ou dépigmentants, dépilatoires, anti-repousse, ou favorisant la pousse des poils et/ou cheveux, peptides, vitamines.

10. Utilisation topique cosmétique d'un matériel d'origine végétale obtenu à partir d'*Oxydendron arboreum* ou un matériel selon l'une quelconques des revendications 1 à 3, pour améliorer l'apparence et l'état général de la peau des muqueuses et des phanères, et notamment pour lutter contre le vieillissement et/ou les imperfections de la peau des muqueuses ou des phanères.

11. Utilisation selon la revendication 11 **caractérisée en ce que** le vieillissement est chronologique, hormonal, actinique (héliodermie) ou dû à des effets environnementaux.

12. Utilisation selon l'une quelconque des revendications 10 ou 11, **caractérisée en ce que** l'amélioration de l'apparence et de l'état général est l'amélioration du teint de la peau, de la radiance, de la fermeté de la peau, une diminution des rides et ridules, de la sécheresse cutanée, du déséquilibre du cuir chevelu, de la perte des cheveux, des ongles cassants.

13. Utilisation selon l'une quelconque des revendications 10 à 12, **caractérisée en ce que** la peau est une peau sensible, sensibilisées, ou réactive.

14. Composition comprenant un matériel selon l'une quelconque des revendications 1 à 3, pour un traitement thérapeutique de la peau, des muqueuses et des phanères dégradés.
